# EUROPEAN PATENT SPECIFICATION

(11) **EP 3 679 026 B1**
(45) Date of publication and mention of the grant of the patent: **02.12.2026**
(21) Application number: 18762092.7
(22) Date of filing: 03.09.2018
(51) Int. Cl.: C07D 401/12, A61K 31/45, A61K 31/4523, C07D 405/12, C07D 409/12, C07D 487/04, C07D 211/88, C07D 495/14, C07D 519/00

(54) **GLUTARIMIDE**
GLUTARIMID
GLUTARIMIDE

(30) Priority: 04.09.2017 EP 17189231
(43) Date of publication of application: 15.07.2020
(73) Proprietor: C4 Therapeutics, Inc., Watertown, MA 02472 (US)
(72) Inventor: GOERGLER, Annick, 4070 Basel (CH); NORCROSS, Roger, 4070 Basel (CH); SCHMID, Philipp, 4070 Basel (CH); DEY, Fabian, 4070 Basel (CH); KUSZNIR, Eric Andre, 4070 Basel (CH)
(74) Representative: Bassil, Nicholas Charles
(86) International application number: PCT/EP2018/073578
(87) International publication number: WO 2019/043214

(56) References cited:
- WO-A1-2008/007979
- WO-A1-2017/197051
- CN-A- 1 696 127
- US-A- 4 558 057
- REGISTRY, CHEMICAL ABSTRACTS SERVICE, COLUMBUS, OHIO, US, 22 July 2016 (2016-07-22), XP002780333
- REGISTRY, CHEMICAL ABSTRACTS SERVICE, COLUMBUS, OHIO, US, 8 July 2016 (2016-07-08), XP002780334
- REGISTRY, CHEMICAL ABSTRACTS SERVICE, COLUMBUS, OHIO, US, 10 February 2014 (2014-02-10), XP002780341
- REGISTRY, CHEMICAL ABSTRACTS SERVICE, COLUMBUS, OHIO, US, 23 October 2013 (2013-10-23), XP002780348
- BUCKNER J C ET AL: "PHASE II STUDY OF ANTINEOPLASTONS A10 (NSC 648539) AND AS2-1 (NSC620261) IN PATIENTS WITH RECURRENT GLIOMA", MAYO CLINIC PROCEEDINGS, DOWDEN HEALTH MEDIA, INC, US, vol. 74, no. 2, 1 February 1999 (1999-02-01), pages 137 - 145, XP000884569, ISSN: 0025-6196, DOI: 10.4065/74.2.137
- DATABASE CA [online] CHEMICAL ABSTRACTS SERVICE, COLUMBUS, OHIO, US; HUANG, JUNQIN ET AL: "Chemical modification of antineoplaston A10 and antitumor activity of its analogs", XP002778549, retrieved from STN Database accession no. 1994:680507
- HUANG, JUNQIN ET AL: "Chemical modification of antineoplaston A10 and antitumor activity of its analogs", ZHONGGUO YIYAO GONGYE ZAZHI , 24(10), 437-41, 451 CODEN: ZYGZEA; ISSN: 1001-8255, 1993
- DATABASE CA [online] CHEMICAL ABSTRACTS SERVICE, COLUMBUS, OHIO, US; XU, WENFANG ET AL: "Synthesis and antitumor activity of glutamine derivatives", XP002778550, retrieved from STN Database accession no. 1993:102407
- XU, WENFANG ET AL: "Synthesis and antitumor activity of glutamine derivatives", ZHONGGUO YIYAO GONGYE ZAZHI , 23(6), 255-8 CODEN: ZYGZEA; ISSN: 1001-8255, 1992
- DATABASE CA [online] CHEMICAL ABSTRACTS SERVICE, COLUMBUS, OHIO, US; HENDRY, L. B. ET AL: "Stereochemical modelling studies of the interaction of antineoplaston A10 with DNA", XP002778551, retrieved from STN Database accession no. 1987:611519
- HENDRY, L. B. ET AL: "Stereochemical modelling studies of the interaction of antineoplaston A10 with DNA", DRUGS UNDER EXPERIMENTAL AND CLINICAL RESEARCH , 13(SUPPL. 1), 77-81 CODEN: DECRDP; ISSN: 0378-6501, 1987

## Description

### Field of the Invention

The present invention provides compounds which bind to the ubiquitously expressed E3 ligase protein cereblon (CRBN) and alter the substrate specificity of the CRBN E3 ubiquitin ligase complex, resulting in breakdown of intrinsic downstream proteins. Present compounds are thus useful for the treatment of various cancers.

### Background of the Invention

The field of targeted protein degradation promoted by small molecules has been intensively studied over the last years¹.

Protein degradation plays a role in various cellular functions, i.e. the concentrations of regulatory proteins are adjusted through degradation into small peptides to maintain health and productivity of the cells.

Cereblon is a protein that forms an E3 ubiquitin ligase complex, which ubiquinates various other proteins. Cereblon is known as primary target for anticancer thalidomide analogs. A higher expression of cereblon has been linked to the efficiency of thalidomide analogs in cancer therapy.

In recent years, a few bifunctional compounds have been described as useful modulators of targeted ubiquitination, e.g. WO2013020557 ² , WO2013063560 ³ , WO 2013106643 ⁴ , WO2015160845⁵, WO2016011906⁶, WO2016105518⁷, WO2017007612⁸, WO2017024318⁹ and WO2017117473¹⁰.

US4558057¹¹, CN1696127¹², Buckner *et al.¹³,* Huang *et al¹⁴.,* Xu *et al.¹⁵,* Hendry *et al¹⁶.,* WO2008007979¹⁷, CAS No. 1491860-78-6¹⁸, CAS No. 1480842-08-7¹⁹, CAS No. 1466257-08-8²⁰, CAS No. 1465134-13-7²¹, CAS No. 1462991-23-6²², WO2017197051²³, CAS No. 1487722-61-1²⁴, CAS No. 1925250-20-9²⁵, CAS No. 1512181-14-4²⁶, CAS No. 1466882-97-2²⁷, CAS No. 1468435-76-8²⁸, CAS No. 1467495-41-5²⁹, CAS No. 1480018-59-4³°, CAS No. 1496032-76-8³¹, CAS No. 1993422-50-6³², CAS No. 1869440-20-9³³, CAS No. 1495663-98-3³⁴, CAS No. 1491860-78-6³⁵, CAS No. 1485498-58-5³⁶, CAS No. 1702002-20-7³⁷, CAS No. 1490092-27³⁸, CAS No. 1540138-47-3 ³⁹, CAS No. 1998850-87-5 ⁴⁰, CAS No. 1966549-05-2 ⁴¹, CAS No. 1957941-94-4⁴², CAS No. 1948185-02-1⁴³, CAS No. 1925097-89-7⁴⁴, CAS No. 1922676-64-9⁴⁵, CAS No. 1923778-80-6⁴⁶, CAS No. 1560748-55-1⁴⁷, CAS No. 1559709-03-3⁴⁸, CAS No. 1543949-70-7⁴⁹, CAS No. 1541076-23-6⁵⁰, CAS No. 1522124-94-2⁵¹, CAS No. 1519516-43-8⁵², CAS No. 1492524-17-0⁵³, CAS No. 1491481-66-3⁵⁴, CAS No. 1486311-15-2⁵⁵, CAS No. 1467658-22-5⁵⁶, CAS No. 1462942-69-3⁵⁷, CAS No. 1464846-46-5⁵⁸, and CAS No. 1466763-16-5⁵⁹ describe certain structurally similar compounds.

However, there is still an ongoing need for effective treatment of cancers.

### Summary of the Invention

The present invention provides a compound of formula I, or a pharmaceutically acceptable salt thereof, 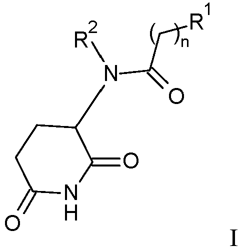 wherein
R¹ is selected from the group consisting of 1,2,3,4-tetrahydroquinolinyl, 1H-indolyl, 2,3-dihydro-1H-pyrrolo[2,3-b]pyridinyl, 6,7-dihydro-5H-cyclopenta[b]pyridinyl, benzofuranyl, benzothiophenyl, chromanyl, indolinyl, and isoindolinyl; optionally substituted by one or more R⁶,
R² is H, C₁₋₆alkyl or -NC(=O)-C₁₋₆alkyl,
R⁶ is H, C₁₋₆alkyl, -C(=O)O-C₁₋₆alkyl or oxo,
n is 0 or 1;
for use as therapeutically active substance.

The present invention further provides a compound of formula I, or a pharmaceutically acceptable salt thereof, 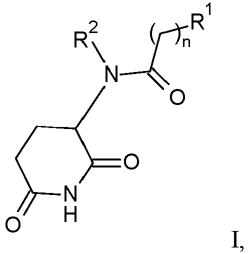 wherein
R¹ is 2,3-dihydro-1H-pyrrolo[2,3-b]pyridinyl, optionally substituted by one or more R⁶;
R² is H or C₁₋₆alkyl,
R⁶ is H or C₁₋₆alkyl,
n is 0 or 1.

The present compounds are useful for the therapeutic and/or prophylactic treatment of cancer.

The compounds of present invention can further be used as part of bifunctional compounds that comprise the compounds of present invention as E3 Ubiquitin Ligase moiety that is linked to a moiety that binds to a target protein where the target protein is proximate to the ubiquitin ligase to effect degradation of said protein.

### Detailed Description of the Invention

The present invention provides a compound of formula I and their pharmaceutically acceptable salts thereof, the preparation of the above mentioned compounds, medicaments containing them and their manufacture as well as the use of the above mentioned compounds in the therapeutic and/or prophylactic treatment of cancer.

The following definitions of the general terms used in the present description apply irrespectively of whether the terms in question appear alone or in combination with other groups.

Unless otherwise stated, the following terms used in this application, including the specification and claims, have the definitions given below. It must be noted that, as used in the specification and the appended claims, the singular forms "a", "an," and "the" include plural referents unless the context clearly dictates otherwise.

The term "C₁₋₆-alkyl", alone or in combination with other groups, stands for a hydrocarbon radical which may be linear or branched, with single or multiple branching, wherein the alkyl group in general comprises 1 to 6 carbon atoms, for example, methyl (Me), ethyl (Et), propyl, isopropyl (i-propyl), n-butyl, i-butyl (isobutyl), 2-butyl (sec-butyl), t-butyl (tert-butyl), isopentyl, 2-ethyl-propyl (2-methyl-propyl), 1,2-dimethyl-propyl and the like. A specific group is methyl.

The term "halogen-C₁₋₆-alkyl", alone or in combination with other groups, refers to C₁₋₆-alkyl as defined herein, which is substituted by one or multiple halogen, particularly 1-5 halogen, more particularly 1-3 halogen. Particular halogen is fluoro. Particular "halogen-C₁₋₆-alkyl" is fluoro-C₁₋₆-alkyl and a particular "halogen-C₁₋₃-alkyl" is fluoro-C₁₋₃-alkyl. Examples are trifluoromethyl, difluoromethyl, fluoromethyl and the like.

The term "hydroxy-C₁₋₆-alkyl", alone or in combination with other groups, refers to C₁₋₆-alkyl as defined herein, which is substituted by one or multiple hydroxy, particularly by 1 hydroxy. Examples are -CH₂OH, -CH₂CH₂OH and the like.

The term "hydroxy", alone or in combination with other groups, refers to OH.

The term "halogen", alone or in combination with other groups, denotes chloro (Cl), iodo (I), fluoro (F) and bromo (Br). A specific group is F.

The term "heteroaryl" denotes a monovalent heterocyclic mono- or bicyclic ring system of 5 to 12 ring atoms, comprising 1, 2, 3 or 4 heteroatoms selected from N, O and S, the remaining ring atoms being carbon and in which at least one ring is aromatic. Examples of heteroaryl moieties include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolinyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, dihydroquinolyl, dihydropyrrolopyridinyl, dihydronaphthyridinyl, chromanyl, tetrahydroquinolinyl, dihydrocyclopentapyridinyl quinazolinyl, or quinoxalinyl.

The term "heterocycloalkyl" denotes a monovalent saturated or partly unsaturated mono- or bicyclic ring system of 4 to 9 ring atoms, comprising 1, 2, or 3 ring heteroatoms selected from N, O and S, the remaining ring atoms being carbon. Examples for monocyclic saturated heterocycloalkyl are azetidinyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl, or dihydropyranyl.

The term "C₁₋₆-alkoxy", alone or in combination with other groups, stands for an -O-C₁₋₆-alkyl radical which may be linear or branched, with single or multiple branching, wherein the alkyl group in general comprises 1 to 6 carbon atoms, for example, methoxy (OMe, MeO), ethoxy (OEt), propoxy, isopropoxy (i-propoxy), n-butoxy, i-butoxy (iso-butoxy), 2-butoxy (sec-butoxy), t-butoxy (tert-butoxy), isopentyloxy (i-pentyloxy) and the like. Particular "C₁₋₆-alkoxy" are groups with 1 to 4 carbon atoms. A specific group is methoxy.

The term "aryl" denotes a monovalent aromatic carbocyclic mono- or bicyclic ring system comprising 6 to 10 carbon ring atoms and in which at least one ring is aromatic. Examples of aryl moieties include phenyl (Ph), indanyl, tetralinyl and naphthyl. Specific "aryl" is phenyl.

Terms like "a-b-x substituted by R" means that the "x" portion of the moiety is substituted by R.

The term "pharmaceutically acceptable" denotes an attribute of a material which is useful in preparing a pharmaceutical composition that is generally safe, non-toxic, and neither biologically nor otherwise undesirable and is acceptable for veterinary as well as human pharmaceutical use.

The term "a pharmaceutically acceptable salt" refers to a salt that is suitable for use in contact with the tissues of humans and animals. Examples of suitable salts with inorganic and organic acids are, but are not limited to acetic acid, citric acid, formic acid, fumaric acid, hydrochloric acid, lactic acid, maleic acid, malic acid, methane-sulfonic acid, nitric acid, phosphoric acid, p-toluenesulphonic acid, succinic acid, sulfuric acid (sulphuric acid), tartaric acid, trifluoroacetic acid and the like. Particular acids are formic acid, trifluoroacetic acid and hydrochloric acid. Specific acids are hydrochloric acid, trifluoroacetic acid and fumaric acid.

The terms "pharmaceutically acceptable auxiliary substance" refer to carriers and auxiliary substances such as diluents or excipients that are compatible with the other ingredients of the formulation.

The term "pharmaceutical composition" encompasses a product comprising specified ingredients in pre-determined amounts or proportions, as well as any product that results, directly or indirectly, from combining specified ingredients in specified amounts. Particularly it encompasses a product comprising one or more active ingredients, and an optional carrier comprising inert ingredients, as well as any product that results, directly or indirectly, from combination, complexation or aggregation of any two or more of the ingredients, or from dissociation of one or more of the ingredients, or from other types of reactions or interactions of one or more of the ingredients.

"Therapeutically effective amount" means an amount of a compound that, when administered to a subject for treating a disease state, is sufficient to effect such treatment for the disease state. The "therapeutically effective amount" will vary depending on the compound, disease state being treated, the severity or the disease treated, the age and relative health of the subject, the route and form of administration, the judgment of the attending medical or veterinary practitioner, and other factors.

The term "as defined herein" and "as described herein" when referring to a variable incorporates by reference the broad definition of the variable as well as particularly, more particularly and most particularly definitions, if any.

The terms "treating", "contacting" and "reacting" when referring to a chemical reaction means adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product.

The term "aromatic" denotes the conventional idea of aromaticity as defined in the literature, in particular in IUPAC - Compendium of Chemical Terminology, 2nd Edition, A. D. McNaught & A. Wilkinson (Eds). Blackwell Scientific Publications, Oxford (1997).

The term "pharmaceutically acceptable excipient" denotes any ingredient having no therapeutic activity and being non-toxic such as disintegrators, binders, fillers, solvents, buffers, tonicity agents, stabilizers, antioxidants, surfactants or lubricants used in formulating pharmaceutical products.

Whenever a chiral carbon is present in a chemical structure, it is intended that all stereoisomers associated with that chiral carbon are encompassed by the structure as pure stereoisomers as well as mixtures thereof.

The invention also provides pharmaceutical compositions, methods of using, and methods of preparing the aforementioned compounds.

All separate embodiments may be combined.

The compounds of formula I may be prepared in accordance with the schemes described in the examples. The starting material is commercially available or may be prepared in accordance with known methods.

The preparation of compounds of formula I is further described in more detail in the schemes below.

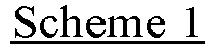

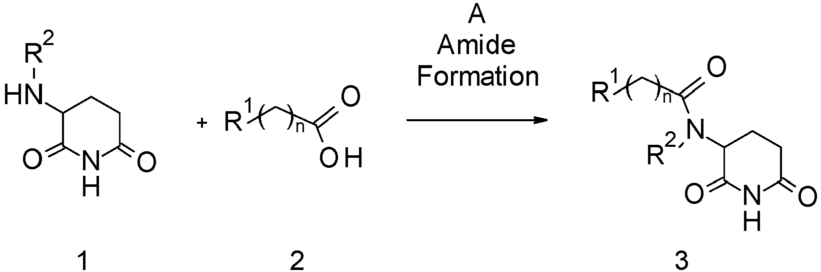

The substituents and n are as described herein.

*Step A:* Amide bond formation can be accomplished by a coupling reaction between a carboxylic acid 2 and a suitable amino-imide 1 in the presence of a coupling reagent such as DCC, EDC, TBTU or HATU in the presence of an organic base such as triethylamine, *N,N-*diisopropylethylamine or N-methylmorpholine in halogenated solvents such as dichloromethane or 1,2-dichloroethane or ethereal solvents such as diethyl ether, dioxane, THF, DME or TBME or polar non-protic organic solvent such as N,N-dimethylformamide at room temperature or at elevated temperatures for 2-18 hours.

Examples of suitable amino-imides 1 include, but are not limited to, 3-aminopiperidine-2,6-dione ⁶⁰ , (3*S*)-3-aminopiperidine-2,6-dione ⁶¹ , (3*S*)-3-aminopiperidine-2,6-dione hydrochloride ⁶² , (3R)-3-aminopiperidine-2,6-dione ⁶³ , (3R)-3-aminopiperidine-2,6-dione hydrochloride⁶⁴, or 3-(methylamino)piperidine-2,6-dione⁶⁵.

Preferred conditions are HATU with N,N-diisopropylethylamine in *N,N-*dimethylformamide at room temperature for 18 hours.

In cases where a racemic mixture of amino-imide 1 is coupled to an achiral carboxylic acid 2, the coupling reaction affords amide 3 as a racemic mixture which may be separated into its constituent enantiomers using chiral HPLC. In cases where the carboxylic acid 2 contains one or more stereogenic centres, the coupling reaction affords amide 3 as a mixture of diastereomers which may be separated using chiral HPLC. 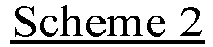 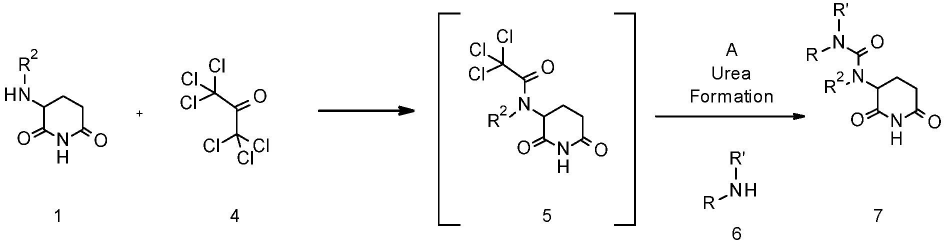 R,R' = H, heterocycloalkyl, heteroaryl or form together with the nitrogen they are attached to a heterocycloalkyl or a heteroaryl R² = as described herein

*Step A:* Urea formation can be accomplished by a coupling reaction between a suitable amino-imide 1, triphosgene 4, and a primary or secondary amine 6. The reaction is carried out in a stepwise manner, involving initial reaction of amino-imide 1 and one equivalent of triphosgene 4 to afford intermediate trichloroacetamide 5 which is then reacted *in situ* with a primary or secondary amine 6 to afford urea 7. The reaction is carried out in the presence of an organic base such as triethylamine, *N*,*N*-diisopropylethylamine or *N*-methylmorpholine in halogenated solvents such as dichloromethane or 1,2-dichloroethane or ethereal solvents such as diethyl ether, dioxane, THF, DME or TBME.

Preferred conditions are triethylamine in 1,2-dichoroethane. The first step to form intermediate 5 is preferably carried out at a temperature between 0 °C and room temperature for 1-2 hours, and the second step to form urea 7 is preferably carried out at room temperature for 18 hours.

Examples of suitable amino-imides 1 include, but are not limited to, 3-aminopiperidine-2,6-dione⁶⁰, (3*S*)-3 -aminopiperidine-2,6-dione⁶¹, (3S)-3-aminopiperidine-2,6-dione hydrochloride⁶², (3*R*)-3-aminopiperidine-2,6-dione⁶³, (3R)-3-aminopiperidine-2,6-dione hydrochloride⁶⁴, or 3-(methylamino)piperidine-2,6-dione⁶⁵.

In cases where a racemic mixture of amino-imide 1 is coupled to an achiral amine 6, the coupling reaction affords urea 7 as a racemic mixture which may be separated into its constituent enantiomers using chiral HPLC. In cases where the amine 6 contains one or more stereogenic centres, the coupling reaction affords urea 7 as a mixture of diastereomers which may be separated using chiral HPLC. 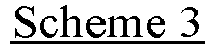 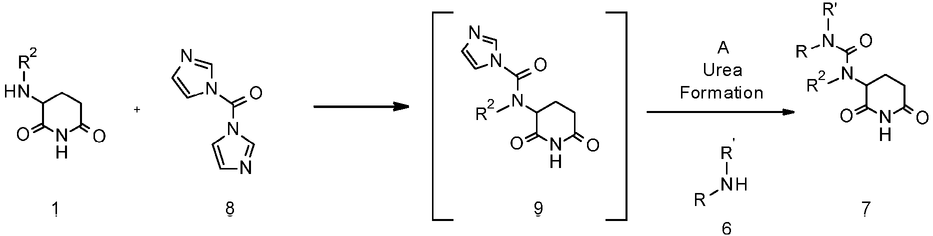 R,R' = H, heterocycloalkyl, heteroaryl or form together with the nitrogen to which they are attached a heterocycloalkyl or a heteroaryl R² = as described herein

*Step A:* Urea formation can also be accomplished by a coupling reaction between a suitable amino-imide 1, 1,1'-carbonyl-di-(1*H*-imidazole) 4, and a primary or secondary amine 6. The reaction is carried out in a stepwise manner, involving initial reaction of amino-imide 1 and 1,1'-carbonyl-di-(1H-imidazole) 4 to afford intermediate imidazole-1-carboxamide 9 which is then reacted *in situ* with a primary or secondary amine 6 to afford urea 7. The reaction is carried out in polar aprotic organic solvent such as N-methyl-2-pyrrolidinone or N,N-dimethylformamide, preferably N,N-dimethylformamide. The first step to form intermediate 9 is preferably carried out at room temperature for 1-2 hours, and the second step to form urea 7 is preferably carried out at 80 °C for 3 hours.

Examples of suitable amino-imides 1 include, but are not limited to, 3-aminopiperidine-2,6-dione⁶¹, (3*S*)-3-aminopiperidine-2,6-dione⁶¹, (3*S*)-3-aminopiperidine-2,6-dione hydrochloride⁶², (3*R*)-3-aminopiperidine-2,6-dione⁶³, (3R)-3-aminopiperidine-2,6-dione hydrochloride⁶⁴, or 3-(methylamino)piperidine-2,6-dione⁶⁵.

In cases where a racemic mixture of amino-imide 1 is coupled to an achiral amine 6, the coupling reaction affords urea 7 as a racemic mixture which may be separated into its constituent enantiomers using chiral HPLC. In cases where the amine 6 contains one or more stereogenic centres, the coupling reaction affords urea 7 as a mixture of diastereomers which may be separated using chiral HPLC.

### Isolation and purification of the compounds

Isolation and purification of the compounds and intermediates described herein can be effected, if desired, by any suitable separation or purification procedure such as, for example, filtration, extraction, crystallization, column chromatography, thin-layer chromatography, thick-layer chromatography, preparative low or high-pressure liquid chromatography or a combination of these procedures. Specific illustrations of suitable separation and isolation procedures can be had by reference to the preparations and examples herein below. However, other equivalent separation or isolation procedures could, of course, also be used. Racemic mixtures of chiral compounds of formula I can be separated using chiral HPLC. Racemic mixtures of chiral synthetic intermediates may also be separated using chiral HPLC.

### Salts of compounds of formula I

In cases where the compounds of formula I are basic they may be converted to a corresponding acid addition salt. The conversion is accomplished by treatment with at least a stoichiometric amount of an appropriate acid, such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like. Typically, the free base is dissolved in an inert organic solvent such as diethyl ether, ethyl acetate, chloroform, ethanol or methanol and the like, and the acid added in a similar solvent. The temperature is maintained between 0 °C and 50 °C. The resulting salt precipitates spontaneously or may be brought out of solution with a less polar solvent.

Insofar as their preparation is not described in the examples, the compounds of formula I as well as all intermediate products can be prepared according to analogous methods or according to the methods set forth herein. Starting materials are commercially available, known in the art or can be prepared by methods known in the art or in analogy thereto.

It will be appreciated that the compounds of general formula I in this invention may be derivatised at functional groups to provide derivatives which are capable of conversion back to the parent compound *in vivo.*

### Pharmacological Tests

The compounds of formula I and their pharmaceutically acceptable salts possess valuable pharmacological properties. The compounds were investigated in accordance with the test given hereinafter.

### Dual Fluorescent Reporter Assay

In order to measure BRD4 protein abundance in a mammalian cell system at medium throughput, a dual fluorescent reporter system was developed based on a principle described in ¹. Transient expression vectors were designed that contain the BRD4 coding sequence (NM_058243.2) fused to a fluorescent tag. Vectors were synthesized at ATUM (Newark, CA, USA) using the pD2610 CMV backbone and were built up as follows: c-terminal version BRD4_eGFP - IRES - FresnoRFP_NLS, n-terminal version eGFP_BRD4 - IRES - PresnoRFP_NLS, empty vector control eGFP - IRES - PresnoRFP_NLS. The c-terminal version was used for the reporter assays, as it presented with the best assay window. HEK293A cells (Invitrogen, Cat. No. R705-07) were cultured in Dulbecco's Modified Eagle Medium (DMEM), 10% fetal calf serum, 2mM L-Glutamine, 1% Penicillin/Streptomycin. Transfections of the plasmids were performed with Lipofectamine 2000 according to the manufacturer's protocol (Invitrogen, Carlsbad, CA, USA). 40 hours after transfection, cells were seeded at a density of 40'000 / 100ul / 96well flat-bottom and 8 hours later treated with compounds (stocks 10mM in DMSO) at a 10-point dilution ranging from 0 - 25 µM. After 16 hours of treatment, cells were washed with PBS, resuspended in Accumax solution (Sigma-Aldrich Cat. No. A7089) and analyzed by flow-cytometry (CytoFlex S, BeckmanCoulter). Single cells were gated based on their forward and side-scatter profiles and pulse-width was used to exclude doublets. A minimum of 20'000 cells was acquired per sample. Analysis was performed with the program Flow Jo V10.1 on BRD4-eGFP low/medium cells (< 10⁶ FL1-A Mean Fluorescence Intensity (MFI)). A factor was derived to normalize BRD4-eGFP values to the RFP protein abundance control (20 x FL1A-GFP / FL11A-RFP), then Median and Mode values were calculated and used for comparisons between treatment conditions.

### Capillary-based Immunoassays to measure endogenous BRD4 levels

The biological activity of selected compounds (cut-off > 20% reduction in BRD4-eGFP levels) was confirmed in an additional assay which allowed the quantification of endogenous BRD4 levels. To this end, HEK293A cells (origin and culture conditions see above) were seeded at 400'000 / 300ul / 48 well and were treated 6 hours later with compound concentrations as indicated for. 16 hours after the treatment, the cells were washed with PBS and lysed in 50ul of UREA lysis buffer (10 mM Tris-HCl pH 8, 2% CHAPS, 7M UREA, 0,4% DTT), supplemented with 1 × protease inhibitor cocktail (Complete Mini, Roche) and 1 x phosphatase inhibitor cocktail (PhosSTOP, Sigma-Aldrich). Samples were then analyzed by Peggy Sue or WES capillary-based immunoassay systems according to the manufacturer's protocol (Protein Simple / Bio-Techne, San Jose, California, 95134 USA). Antibodies used were anti- BRD4 (Cell signaling, CST 13440 1:50) and anti-Vinculin (Sigma, V9131, 1:4000). To quantify BRD4 protein levels, the peak signal areas were normalized to the vinculin loading control and to the DMSO condition.

Further, please see Yen *et al.*⁷¹.

### Fluorescence Direct Binding Protocol

### Principle

Determination of the affinities of compounds to protein containing one or more tryptophan is measurable by monitoring the fluorescence emission in direct mode. The measurements depending on the protein available amounts are performed either manually in a cuvette on ISS-PC1 photon counting spectrofluorometer or automatically in well plates on a fluorescence plate reader device. Fluorescence titrations are performed at 20 °C in the chosen binding assay buffer by using a defined constant protein concentration against ligand concentration variations. Small aliquots of known ligand concentration solubilized in DMSO were added and the fluorescence, excited at 280 nm, was recorded at 340 nm. The fluorescence intensity was corrected for protein dilution and for the filter effect (Birdsall *et al.*⁷²). The corrected fluorescence intensity was plotted against the ligand concentration and fitted using a four-parameter sigmoidal function, from which the equilibrium dissociation constant Kd was computed using the law of mass action assuming a 1:1 protein-ligand complex (Eftink, 1997⁷³).

### Process

1) Optimization of measurement parameters to minimize protein consumption and to minimize the dilution effect and the DMSO content
2) Titration measurements of the protein against ligand by at least 12 titration steps to obtain a good s-curve fit
3) Repeat the same titration measurements with the ligand alone to enable correction
4) Check the stability of the protein once by titration against DMSO alone
5) Determination of the molar extinction coefficients of the ligand at 280 and 340 nm with help of an UV- spectrophotometer
6) Use Excel template for the correction of the measured raw data
7) Use GraphPad Prism software for the quadratic binding fit and the K_{D} evaluation.

### Experimental Details

**Table 1: Protein - buffers, Reference compound: thalidomide, Contergan, Softenon**

| **Protein Batch #** | **Cereblon_17_13** |
|---|---|
| Construct name | hCereblon(M1-L442)_hDDB1(M1-H1140) |
| Concentration | 2.54 mg/ml |
| MW | 180180 Da |
| Molar extinction coefficient | ε₂₈₀ = 165045 M⁻¹.cm⁻¹ |
| Storage buffer | 20 mM MES pH 6.5 200 mM NaCl 1 mM TCEP |
| Assay buffer | 50 mM Hepes 7.4 200 mM NaCl |

**Table 2: Settings**

| Device | ISS-PC1 |
|---|---|
| Excitation wavelength [nm] | 280 |
| Emission wavelength [nm] | 340 |
| Cuvette | Hellma 115F-QS |
| Volume [µL] | 500 |

**Table 3 : Protein preparation**

| Protein preparation : | | |
|---|---|---|
| Volume Protein [µL] | Volume buffer [µL] | Protein concentration [M] |
| 1.8 @ 2.54 mg/ml | 498.2 | 5.0 E-8 |

**Table 4: Titration steps**

| **C Lig [ M ]** | **C Aliquot [ M ]** | **V Aliquot [ µL ]** | **C Prot[ M ]** | **Dilution factor** |
|---|---|---|---|---|
| 1E-10 | 1.0E-07 | 0.5 | 4.995E-08 | 1.001 |
| 1.1E-09 | 1.0E-06 | 0.5 | 4.990E-08 | 1.002 |
| 3.1E-09 | 1.0E-06 | 1 | 4.980E-08 | 1.004 |
| 5.1E-09 | 1.0E-06 | 1 | 4.970E-08 | 1.006 |
| 1.51E-08 | 1.0E-05 | 0.5 | 4.965E-08 | 1.007 |
| 2.51E-08 | 1.0E-05 | 0.5 | 4.960E-08 | 1.008 |
| 4.51E-08 | 1.0E-05 | 1 | 4.950E-08 | 1.01 |
| 6.51E-08 | 1.0E-05 | 1 | 4.941E-08 | 1.012 |
| 1.651E-07 | 1.0E-04 | 0.5 | 4.936E-08 | 1.013 |
| 3.651E-07 | 1.0E-04 | 1 | 4.926E-08 | 1.015 |
| 5.651E-07 | 1.0E-04 | 1 | 4.916E-08 | 1.017 |
| 7.651E-07 | 1.0E-04 | 1 | 4.907E-08 | 1.019 |
| 9.651E-07 | 1.0E-04 | 1 | 4.897E-08 | 1.021 |
| 1.9651E-06 | 1.0E-03 | 0.5 | 4.892E-08 | 1.022 |
| 2.9651E-06 | 1.0E-03 | 0.5 | 4.888E-08 | 1.023 |
| 1.29651E-05 | 1.0E-02 | 0.5 | 4.883E-08 | 1.024 |
| 2.29651E-05 | 1.0E-02 | 0.5 | 4.878E-08 | 1.025 |
| 4.29651E-05 | 1.0E-02 | 1 | 4.869E-08 | 1.027 |
| 6.29651E-05 | 1.0E-02 | 1 | 4.859E-08 | 1.029 |
| 8.29651E-05 | 1.0E-02 | 1 | 4.850E-08 | 1.031 |

**Table 5: affinities of examples to protein**

| **Example** | **Name** | **Fluorescence h-Cereblon_DDB1 Mean K_{d}_EQ (µM)** |
|---|---|---|
| 1 | *N*-[(3*S*)-2,6-dioxo-3-piperidyl]-2-phenyl-acetamide | 0.027 |
| 2 | *N*-(2,6-dioxo-3-piperidyl)indane-1-carboxamide | 0.655 |
| 3 | *N*-(2,6-dioxo-3-piperidyl)-2-propyl-isoindoline-1-carboxamide | 0.057 |
| 4 | *N*-(2,6-dioxo-3-piperidyl)indane-1-carboxamide, isomer A | 0.068 |
| 5 | *N*-(2,6-dioxo-3-piperidyl)indane-1-carboxamide, isomer B | 0.197 |
| 6 | *N*-(2,6-dioxo-3-piperidyl)indane-1-carboxamide | 0.146 |
| 7 | *N*-(2,6-dioxo-3-piperidyl)-1*H*-indole-3-carboxamide | 0.028 |
| 8 | *N*-(2,6-dioxo-3-piperidyl)-1-methyl-indole-3-carboxamide | 0.030 |
| 9 | *N*-(2,6-dioxo-3-piperidyl)indoline-1-carboxamide | 0.002 |
| 10 | *N*-(2,6-dioxo-3-piperidyl)benzamide | 0.020 |
| 11 | *N*-(2,6-dioxo-3-piperidyl)cyclopentanecarboxamide | 0.012 |
| 12 | *N*-(2,6-dioxo-3-piperidyl)benzofuran-3-carboxamide | 0.002 |
| 13 | *N*-(2,6-dioxo-3-piperidyl)benzothiophene-3-carboxamide | 0.008 |
| 14 | (1*R*)-*N*-(2,6-dioxo-3-piperidyl)tetralin-1-carboxamide | 0.004 |
| 15 | (1*S*)-*N*-(2,6-dioxo-3-piperidyl)tetralin-1-carboxamide | < 0.001 |
| 16 | *N*-(2,6-dioxo-3-piperidyl)-*N*-methyl-1*H*-indole-3-carboxamide | *0.003* |
| 17 | *N*-[(3*S*)-2,6-dioxo-3-piperidyl]-1*H*-indole-3-carboxamide | 0.014 |
| 18 | *N*-[(3*R*)-2,6-dioxo-3-piperidyl]-1*H*-indole-3-carboxamide | 0.014 |
| 19 | *N*-((*S*)-2,6-dioxopiperidin-3-yl)-6,7-dihydro-5*H-*cyclopenta[b]pyridine-5-carboxamide | 0.001 |
| 20 | *N-*((*R*)-2,6-dioxopiperidin-3-yl)-6,7-dihydro-5*H-*cyclopenta[b]pyridine-5-carboxamide | 0.003 |
| 21 | (*S*)-*N*-(2,6-dioxopiperidin-3-yl)benzofuran-3-carboxamide | 0.001 |
| 22 | (*N*-((*R*)-2,6-dioxopiperidin-3-yl)-6,7-dihydro-5*H-*cyclopenta[b]pyridine-7-carboxamide | < 0.001 |
| 23 | *N*-((*S*)-2,6-dioxopiperidin-3-yl)-6,7-dihydro-5*H-*cyclopenta[b]pyridine-7-carboxamide | 0.025 |
| 24 | (*R*)-*N*-(2,6-dioxopiperidin-3-yl)benzo[b]thiophene-3-carboxamide | 0.009 |
| 25 | (*S*)-*N*-(2,6-dioxopiperidin-3-yl)benzo[b]thiophene-3-carboxamide | 0.023 |
| 26 | (*S*)-*N*-((*R*)-2,6-dioxopiperidin-3-yl)-1,2,3,4-tetrahydronaphthalene-1-carboxamide | 0.006 |
| 27 | (*S*)-*N*-((*S*)-2,6-dioxopiperidin-3-yl)-1,2,3,4-tetrahydronaphthalene-1-carboxamide | 0.003 |
| 28 | (*S*)-*N*-(2,6-dioxopiperidin-3-yl)-3,4-dihydroquinoline-1(2H)-carboxamide | 0.004 |
| 29 | (*S*)-*N*-(2,6-dioxopiperidin-3-yl)-2,3-dihydro-1*H-*pyrrolo[2,3-b]pyridine-1-carboxamide | < 0.001 |
| 30 | (*S*)-*N*-(2,6-dioxopiperidin-3-yl)-3,4-dihydro-1,8-naphthyridine-1(2*H*)-carboxamide | 0.004 |
| 31 | *N*-[(3*S*)-2,6-dioxo-3-piperidyl]chromane-4-carboxamide | 0.005 |
| 32 | (4*S*)-*N*-[(3*S*)-2,6-dioxopiperidin-3-yl]-1,2,3,4-tetrahydroquinoline-4-carboxamide | < 0.001 |
| 33 | (4*R*)-*N*-[(3*S*)-2,6-dioxopiperidin-3-yl]-1,2,3,4-tetrahydroquinoline-4-carboxamide | 0.006 |
| 34 | (*R*)-*N*-(2,6-dioxopiperidin-3-yl)benzofuran-3-carboxamide | 0.026 |
| 35 | (*R*)-*N*-(2,6-dioxopiperidin-3-yl)-3,4-dihydroquinoline-1(2*H*)-carboxamide | 0.019 |
| 36 | (*R*)-*N*-(2,6-dioxopiperidin-3-yl)-2,3-dihydro-1*H-*pyrrolo[2,3-b]pyridine-1-carboxamide | 0.010 |
| 37 | *N*-[(3*S*)-2,6-dioxo-3-piperidyl]-1,2,3,4-tetrahydroquinoline-4-carboxamide | < 0.001 |
| 38 | *N*-[(3*S*)-2,6-dioxo-3-piperidyl]-1-methyl-3,4-dihydro-2*H-*quinoline-4-carboxamide | 0.005 |
| 39 | *N*-[(3*R*)-2,6-dioxo-3-piperidyl]-1,2,3,4-tetrahydroquinoline-4-carboxamide | 0.019 |
| 40 | *N*-[(3*R*)-2,6-dioxo-3-piperidyl]-1-methyl-3,4-dihydro-2*H-*quinoline-4-carboxamide | 0.068 |
| 41 | *N*-[(3*R*)-2,6-dioxo-3-piperidyl]chromane-4-carboxamide | 0.014 |
| 42 | methyl (*S*)-3-((2,6-dioxopiperidin-3-yl)carbamoyl)-1*H-*indole-6-carboxylate | 0.018 |
| 43 | (4*S*)-*N*-((3*R*)-2,6-dioxopiperidin-3-yl)-1,2,3,4-tetrahydroquinoline-4-carboxamide | < 0.001 |
| 44 | (4*R*)-*N*-[(3*R*)-2,6-dioxopiperidin-3-yl]-1,2,3,4-tetrahydroquinoline-4-carboxamide | 0.012 |
| 45 | (1*R*)-*N*-((3*R*)-2,6-dioxopiperidin-3-yl)-1,2,3,4-tetrahydronaphthalene-1-carboxamide | 0.004 |
| 46 | (1*R*)-*N*-((3*S*)-2,6-dioxopiperidin-3-yl)-1,2,3,4-tetrahydronaphthalene-1-carboxamide | 0.039 |
| 47 | (*S*)-*N*-(2,6-dioxopiperidin-3-yl)pyrazolo[1,5-a]pyridine-3-carboxamide | < 0.001 |
| 48 | (*R*)-*N*-(2,6-dioxopiperidin-3-yl)pyrazolo[1,5-a]pyridine-3-carboxamide | < 0.001 |
| 49 | (*R*)-*N*-(2,6-dioxopiperidin-3-yl)-1*H*-indazole-3-carboxamide | < 0.001 |
| 50 | (*S*)-*N*-(2,6-dioxopiperidin-3-yl)imidazo[1,5-a]pyridine-3-carboxamide | 0.004 |
| 51 | (*R*)-*N*-(2,6-dioxopiperidin-3-yl)imidazo[1,5-a]pyridine-3-carboxamide | 0.003 |
| 52 | (*S*)-*N*-(2,6-dioxopiperidin-3-yl)indolizine-3-carboxamide | 0.006 |
| 53 | (*R*)-*N*-(2,6-dioxopiperidin-3-yl)indolizine-3-carboxamide | 0.002 |
| 54 | (*S*)-*N*-(2,6-dioxopiperidin-3-yl)imidazo[1,2-a]pyridine-3-carboxamide | 0.001 |
| 55 | (*R*)-*N*-(2,6-dioxopiperidin-3-yl)imidazo[1,2-a]pyridine-3-carboxamide | < 0.001 |
| 56 | (*S*)-*N*-(2,6-dioxopiperidin-3-yl)pyrazolo[1,5-a]pyrimidine-3-carboxamide | 0.008 |
| 57 | (R)-N-(2,6-dioxopiperidin-3-yl)pyrazolo[1,5-a]pyrimidine-3-carboxamide | 0.007 |
| 58 | *N*-(2,6-dioxopiperidin-3-yl)-*N*-methylindoline-1-carboxamide | 0.022 |
| 59 | (*S*)-*N*-(2,6-dioxopiperidin-3-yl)-*N*-methylindoline-1-carboxamide | 0.020 |
| 60 | (*R*)-*N*-(2,6-dioxopiperidin-3-yl)-*N*-methylindoline-1-carboxamide | 0.030 |
| 61 | (*S*)-*N*-(2,6-dioxopiperidin-3-yl)-2-(pyridin-3-yl)acetamide | 0.967 |
| 62 | N-(2,6-dioxopiperidin-3-yl)-*N*-methyl-2,3-dihydro-1*H-*pyrrolo[2,3-b]pyridine-1-carboxamide | 0.029 |
| 63 | (*R*)-*N*-(2,6-dioxopiperidin-3-yl)-2-(pyridin-3-yl)acetamide | 0.019 |
| 64 | (*R*)-*N*-(2,6-dioxopiperidin-3-yl)-3,4-dihydro-1,8-naphthyridine-1(2*H*)-carboxamide | 0.641 |
| 65 | (*S*)-2-(4-acetamidophenyl)-*N*-(2,6-dioxopiperidin-3-yl)acetamide | 0.023 |
| 66 | (*R*)-2-(4-acetamidophenyl)-*N*-(2,6-dioxopiperidin-3-yl)acetamide | 0.086 |
| 67 | (*R*)-2-(3-acetamidophenyl)-*N*-(2,6-dioxopiperidin-3-yl)acetamide | 0.003 |
| 68 | (*S*)-2-(3-acetamidophenyl)-*N*-(2,6-dioxopiperidin-3-yl)acetamide | 0.060 |
| 69 | (*S*)-*N*-(2,6-dioxopiperidin-3-yl)-2-(2-oxoindolin-5-yl)acetamide | 0.015 |
| 70 | (*R*)-*N*-(2,6-dioxopiperidin-3-yl)-2-(2-oxoindolin-5-yl)acetamide | 0.003 |
| 71 | *tert*-butyl (*S*)-3-((2,6-dioxopiperidin-3-yl)carbamoyl)-1-methyl-1,4,6,7-tetrahydro-5*H*-pyrazolo[4,3-c]pyridine-5-carboxylate | 0.004 |
| 72 | (*S*)-*N*-(2,6-dioxopiperidin-3-yl)-1-methyl-4,5,6,7-tetrahydro-1H-pyrazolo[4,3-c]pyridine-3-carboxamide | 0.194 |
| 73 | (1*S*)-*N*-(1-methyl-2,6-dioxopiperidin-3-yl)-1,2,3,4-tetrahydronaphthalene-1-carboxamide | 0.028 |
| 74 | (1*S*)-*N*-(1-methyl-2,6-dioxopiperidin-3-yl)-1,2,3,4-tetrahydronaphthalene-1-carboxamide, isomer A | 0.005 |
| 75 | (1*S*)-*N*-(1-methyl-2,6-dioxopiperidin-3-yl)-1,2,3,4- tetrahydronaphthalene-1-carboxamide, isomer B | 0.046 |

### Pharmaceutical Compositions

The compounds of formula I and the pharmaceutically acceptable salts can be used as therapeutically active substances, e.g. in the form of pharmaceutical preparations. The pharmaceutical preparations can be administered orally, e.g. in the form of tablets, coated tablets, dragées, hard and soft gelatin capsules, solutions, emulsions or suspensions. The administration can, however, also be effected rectally, e.g. in the form of suppositories, or parenterally, e.g. in the form of injection solutions.

The compounds of formula I and the pharmaceutically acceptable salts thereof can be processed with pharmaceutically inert, inorganic or organic carriers for the production of pharmaceutical preparations. Lactose, corn starch or derivatives thereof, talc, stearic acids or its salts and the like can be used, for example, as such carriers for tablets, coated tablets, dragées and hard gelatin capsules. Suitable carriers for soft gelatin capsules are, for example, vegetable oils, waxes, fats, semi-solid and liquid polyols and the like. Depending on the nature of the active substance no carriers are however usually required in the case of soft gelatin capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparations can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula I or a pharmaceutically acceptable salt thereof and a therapeutically inert carrier are also provided by the present invention, as is a process for their production, which comprises bringing one or more compounds of formula I and/or pharmaceutically acceptable salts thereof and, if desired, one or more other therapeutically valuable substances into a galenical administration form together with one or more therapeutically inert carriers.

The dosage can vary within wide limits and will, of course, have to be adjusted to the individual requirements in each particular case. **In** the case of oral administration the dosage for adults can vary from about 0.01 mg to about 1000 mg per day of a compound of general formula I or of the corresponding amount of a pharmaceutically acceptable salt thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical preparations conveniently contain about 1-500 mg, particularly 1-100 mg, of a compound of formula I or formula P-L-C. Examples of compositions according to the invention are:

### Example A

Tablets of the following composition are manufactured in the usual manner:

**Table 6: possible tablet composition**

| ingredient | mg/tablet | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50 °C.
3. Pass the granules through suitable milling equipment.
4. Add ingredient 5 and mix for three minutes; compress on a suitable press.

### Example B-1

Capsules of the following composition are manufactured:

**Table 7: possible capsule ingredient composition**

| ingredient | mg/capsule | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula I lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 8: possible soft gelatin capsule ingredient composition**

| ingredient | mg/capsule |
|---|---|
| Compound of formula I | 5 |
| Yellow wax | 8 |
| Hydrogenated Soya bean oil | 8 |
| Partially hydrogenated plant oils | 34 |
| Soya bean oil | 110 |
| Total | 165 |

**Table 9: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula I is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 10: possible suppository composition**

| ingredient | mg/supp. |
|---|---|
| Compound of formula I | 15 |
| Suppository mass | 1285 |
| Total | 1300 |

### Manufacturing Procedure

The suppository mass is melted in a glass or steel vessel, mixed thoroughly and cooled to 45 °C. Thereupon, the finely powdered compound of formula I is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 11: possible injection solution composition**

| ingredient | mg/injection solution. |
|---|---|
| Compound of formula I | 3 |
| Polyethylene Glycol 400 | 150 |
| acetic acid | q.s. ad pH 5.0 |
| water for injection solutions | ad 1.0 ml |

### Manufacturing Procedure

The compound of formula I is dissolved in a mixture of Polyethylene Glycol 400 and water for injection (part). The pH is adjusted to 5.0 by addition of acetic acid. The volume is adjusted to 1.0 ml by addition of the residual amount of water. The solution is filtered, filled into vials using an appropriate overage and sterilized.

### Example E

Sachets of the following composition are manufactured:

**Table 12: possible sachet composition**

| ingredient | mg/sachet |
|---|---|
| Compound of formula I | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula I is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Experimental Part

The following examples are provided for illustration of the invention.

### Example 1⁷⁴ (provided for reference)

### (S)-N-(2,6-Dioxo-3-piperidinyl)benzeneacetamide

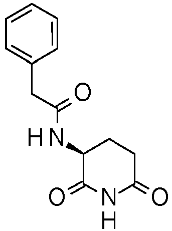

The title compound can be purchased.

### Example 2 (provided for reference)

### N-(2,6-Dioxopiperidin-3-yl)-2,3-dihydro-1H-indene-1-carboxamide (RACEMIC MIXTURE OF ALL DIASTEREOMERS)

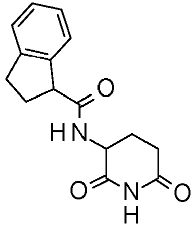

To a stirred suspension of 2,3-dihydro-1H-indene-1-carboxylic acid⁷⁵ (100 mg), *N,N-*diisopropylethylamine (323 µl) and 3-aminopiperidine-2,6-dione⁶⁰ (86.9 mg) in DMF (1.5 ml) was added HATU (352 mg). The reaction mixture was stirred at room temperature for 18 hours. The resulting yellow solution was directly purified by reversed phase HPLC to afford after lyophilisation N-(2,6-dioxopiperidin-3-yl)-2,3-dihydro-1H-indene-1-carboxamide (RACEMIC MIXTURE OF ALL DIASTEREOMERS) as a white solid (168 mg, 66%). MS (ISP): 273.5 ([M+H]⁺).

### Example 3

### N-(2,6-Dioxopiperidin-3-yl)-2-propylisoindoline-1-carboxamide (RACEMIC MIXTURE OF ALL DIASTEREOMERS)

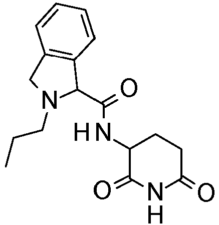

### a) tert-Butyl 1-((2,6-dioxopiperidin-3- yl)carbamoyl)isoindoline-2- carboxylate

The title compound was obtained in analogy to example 2 using (RS)-BOC-1,3-dihydro-2H-isoindole carboxylic acid⁷⁶ in place of 2,3-dihydro-1H-indene-1-carboxylic acid⁷⁵. White solid. MS (ISP): 372.4 ([M-H]⁻).

### b) N-(2,6-Dioxopiperidin-3- yl)isoindoline-1-carboxamide hydrochloride salt.

To a stirred solution of tert-butyl 1-((2,6-dioxopiperidin-3- yl)carbamoyl)isoindoline-2-carboxylate (88 mg) in dichloromethane (2 ml) was added a 4 M solution of HCl in dioxane (884 µl). After stirring for 3 hours, the solvents were evaporated to dryness and diethyl ether was added to the residue. The suspension was sonicated, stirred at 0 °C for 15 min, then filtered to afford N-(2,6-dioxopiperidin-3- yl)isoindoline-1-carboxamide hydrochloride salt as an off-white solid (72 mg, 99%). MS (ISP): 274.1 ([M+H]⁺).

### c) N-(2,6-Dioxopiperidin-3- yl)-2- propylisoindoline-1-carboxamide

To a stirred solution of N-(2,6-dioxopiperidin-3- yl)isoindoline-1-carboxamide hydrochloride salt (63 mg), propionaldehyde⁷⁷ (22.2 µl), sodium acetate (16.7 mg) and zinc chloride (111 mg) in MeOH (1.2 ml) was added sodium cyanoborohydride (38.3 mg). The reaction mixture was stirred at room temperature for 5 hours. The reaction mixture was directly purified by reversed phase HPLC to afford N-(2,6-dioxopiperidin-3- yl)-2- propylisoindoline-1- carboxamide as an white solid (31 mg, 48%). MS (ISP): 316.0 ([M+H]⁺).

### Example 4 (provided for reference)

### (-)-N-(2,6-Dioxopiperidin-3-yl)-2,3-dihydro-1H-indene-1-carboxamide (EPIMER 1)

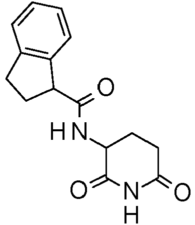

The diastereomers of *N*-(2,6-dioxopiperidin-3-yl)-2,3-dihydro-1*H*-indene-1-carboxamide from example 2 (95 mg) were separated using chiral HPLC (column: Reprosil Chiral-NR, eluent: heptane/ethanol+NH₄OAc (70:30), pressure: 18 bar; flow rate: 35 ml/min) to afford (-)-N-(2,6-dioxopiperidin-3-yl)-2,3-dihydro-1H-indene-1-carboxamide (EPIMER 1) (17 mg, white solid), retention time = 36.1 min. MS (ISP): 273.1 ([M+H]⁺).

### Example 5 (provided for reference)

### (+)-N-(2,6-Dioxopiperidin-3-yl)-2,3-dihydro-1H-indene-1-carboxamide (EPIMER 2)

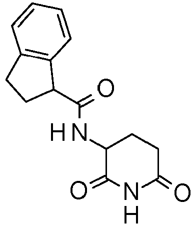

The diastereomers of *N*-(2,6-dioxopiperidin-3-yl)-2,3-dihydro-1*H*-indene-1-carboxamide from example 2 (95 mg) were separated using chiral HPLC (column: Reprosil Chiral-NR, eluent: heptane/ethanol+NH₄OAc (70:30), pressure: 18 bar; flow rate: 35 ml/min) to afford (+)-*N*-(2,6-dioxopiperidin-3-yl)-2,3-dihydro-1H-indene-1-carboxamide (EPIMER 2) (15 mg, white solid), retention time = 45 min. MS (ISP): 273.1 ([M+H]⁺).

### Example 6 (provided for reference)

### N-(2,6-Dioxopiperidin-3-yl)-2,3-dihydro-1H-indene-1-carboxamide (EPIMERS 3 & 4)

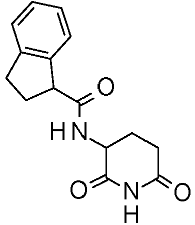

The diastereomers of *N*-(2,6-dioxopiperidin-3-yl)-2,3-dihydro-1*H*-indene-1-carboxamide from example 2 (95 mg) were separated using chiral HPLC (column: Reprosil Chiral-NR, eluent: heptane/ethanol + NH₄OAc (70:30), pressure: 18 bar; flow rate: 35 ml/min) to afford a mixture of two epimers of*N*-(2,6-dioxopiperidin-3-yl)-2,3-dihydro-1*H*-indene-1-carboxamide (EPIMER 3 & 4) (28 mg, white solid), retention time = 63.3 min, MS (ISP): 273.1 ([M+H]⁺).

### Example 7⁷⁸

### N-(2,6-Dioxopiperidin-3-yl)-1H-indole-3-carboxamide

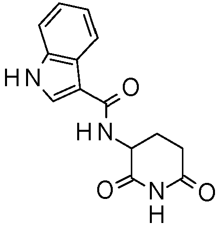

The title compound can be purchased.

### Example 8⁷⁹

### N-(2,6-Dioxopiperidin-3-yl)-1-methyl-1H-indole-3-carboxamide

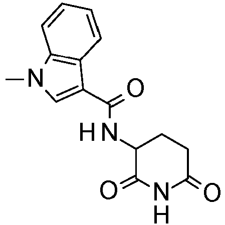

The title compound can be purchased.

### Example 9

### N-(2,6-Dioxopiperidin-3-yl)indoline-1-carboxamide

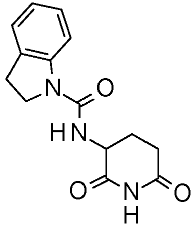

To a stirred suspension of 3-aminopiperidine-2,6-dione⁶⁰ (199 mg) and triethylamine (433 µl) in dichloroethane (12 ml) in a sealed tube was added triphosgene (170 mg) at 0-5 °C. After stirring at room temperature for 1 hour, indoline⁸⁰ (185 mg) was added. The reaction mixture was stirred at room temperature for 18 hours. Some drops of water were added to quench the reaction then the solvent was evaporated to dryness and the residue was purified by reversed phase HPLC (Gemini NX C18, 12 nm, 5 µ, 100 x 30 mm, 40 ml/min, CH₃CN/H₂O + formic acid) to afford, after lyophilisation, N-(2,6-dioxopiperidin-3-yl)indoline-1-carboxamide as a yellow solid (24 mg, 6%). MS (ISP): 274.2 ([M+H]⁺).

### Example 10⁸¹ (provided for reference)

### N-(2,6-Dioxopiperidin-3-yl)benzamide

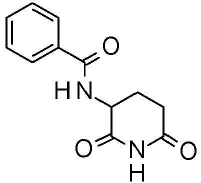

The title compound can be purchased.

### Example 11⁸² (provided for reference)

### N-(2,6-Dioxopiperidin-3-yl)cyclopentanecarboxamide

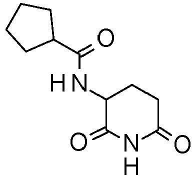

The title compound can be purchased.

### Example 12⁸³

### N-(2,6-Dioxopiperidin-3-yl)benzofuran-3-carboxamide

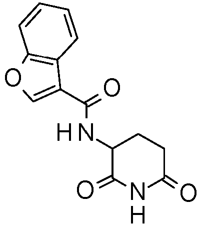

The title compound can be purchased.

### Example 13⁸⁴

### N-(2,6-Dioxopiperidin-3-yl)benzo[b]thiophene-3-carboxamide

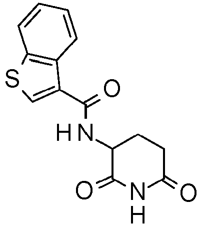

The title compound can be purchased.

### Example 14 (provided for reference)

### (1R)-N-(2,6-Dioxopiperidin-3-yl)-1,2,3,4-tetrahydronaphthalene-1-carboxamide (MIXTURE OF 2 EPIMERS)

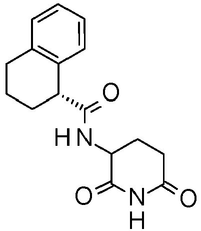

The title compound was obtained in analogy to example 2 using (R)-1,2,3,4-tetrahydronaphthoic acid⁸⁵ in place of 2,3-dihydro-1H-indene-1-carboxylic acid⁷⁵. White solid. MS (ISP): 287 ([M+H]⁺).

### Example 15 (provided for reference)

### (1S)-N-(2,6-Dioxopiperidin-3-yl)-1,2,3,4-tetrahydronaphthalene-1-carboxamide (MIXTURE OF 2 EPIMERS)

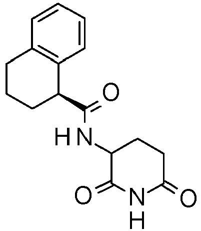

The title compound was obtained in analogy to example 2 using (S)-1,2,3,4-tetrahydronaphthoic acid⁸⁶ in place of 2,3-dihydro-1H-indene-1-carboxylic acid⁷⁵. White solid. MS (ISP): 287 ([M+H]⁺).

### Example 16

### N-(2,6-Dioxopiperidin-3-yl)-N-methyl-1H-indole-3-carboxamide

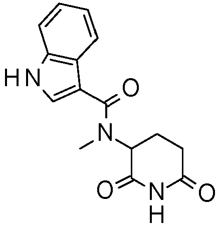

The title compound was obtained in analogy to example 2 using 1H-indole-3-carboxylic acid⁸⁷ in place of 2,3-dihydro-1H-indene-1-carboxylic acid⁷⁵ and 3-(methylamino)piperidine-2,6-dione⁶⁵. in place of 3-aminopiperidine-2,6-dione⁶⁰ . Light blue solid. MS (ISP): 286.2 ([M+H]⁺).

### Example 17

### (S)-N-(2,6-Dioxopiperidin-3-yl)-1H-indole-3-carboxamide

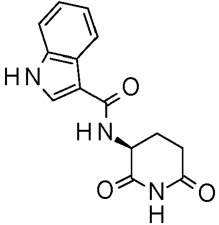

The title compound was obtained in analogy to example 2 using 1H-indole-3-carboxylic acid⁸⁷ in place of 2,3-dihydro-1H-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 272.2 ([M+H]⁺).

### Example 18

### (R)-N-(2,6-Dioxopiperidin-3-yl)-1H-indole-3-carboxamide

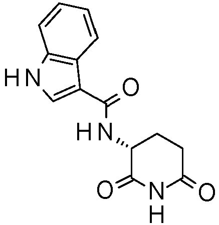

The title compound was obtained in analogy to example 2 using 1H-indole-3-carboxylic acid⁸⁷ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. White solid. MS (ISP):272.2 ([M+H]⁺).

### Example 19

### N-((S)-2,6-Dioxopiperidin-3-yl)-6,7-dihydro-5H-cyclopenta[b]pyridine-5-carboxamide (MIXTURE OF 2 EPIMERS)

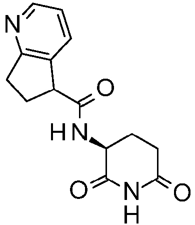

The title compound was obtained in analogy to example 2 using (RS)-6,7-dihydro-5H-cyclopenta[b]pyridine-5-carboxylic acid⁸⁸ in place of 2,3-dihydro-1H-indene-1-carboxylic acid⁷⁵ and (3S)-3-aminopiperidine-2,6-dione hydrochloride⁶² in place of 3-aminopiperidine-2,6-dione⁶⁰. White solid. MS (ISP): 274.3 ([M+H]⁺).

### Example 20

### N-((R)-2,6-Dioxopiperidin-3-yl)-6,7-dihydro-5H-cyclopenta[b]pyridine-5-carboxamide (MIXTURE OF 2 EPIMERS)

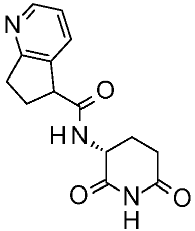

The title compound was obtained in analogy to example 2 using (RS)-6,7-dihydro-5H-cyclopenta[b]pyridine-5-carboxylic acid⁸⁸ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. White solid. MS (ISP): 274.3 ([M+H]⁺).

### Example 21

### (S)-N-(2,6-Dioxopiperidin-3-yl)benzofuran-3-carboxamide

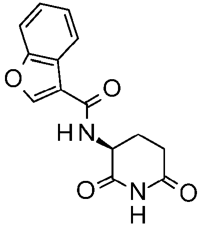

The title compound was obtained in analogy to example 2 using benzofuran-3-carboxylic acid in place of 2,3-dihydro-1H-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 273.2 ([M+H]⁺).

### Example 22 (provided for reference)

### (N-((R)-2,6-Dioxopiperidin-3-yl)-6,7-dihydro-5H-cyclopenta[b]pyridine-7-carboxamide (MIXTURE OF 2 EPIMERS)

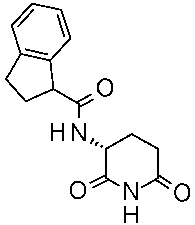

The title compound was obtained in analogy to example 2 using (3R)-3-aminopiperidine-2,6-dione hydrochloride⁶⁴ in place of 3-aminopiperidine-2,6-dione⁶⁰. White solid. MS (ISP): 274.2 ([M+H]⁺).

### Example 23 (provided for reference)

### N-((S)-2,6-Dioxopiperidin-3-yl)-6,7-dihydro-5H-cyclopenta[b]pyridine-7-carboxamide (MIXTURE OF 2 EPIMERS)

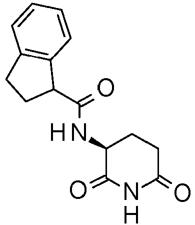

The title compound was obtained in analogy to example 2 using (3*S*)-3-aminopiperidine-2,6-dione hydrochloride⁶² in place of 3-aminopiperidine-2,6-dione⁶⁰. White solid. MS (ISP): 274.2 ([M+H]⁺).

### Example 24

### (R)-N-(2,6-Dioxopiperidin-3-yl)benzo[b]thiophene-3-carboxamide

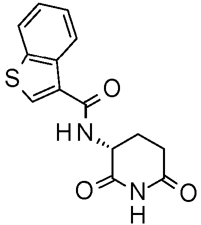

The title compound was obtained in analogy to example 2 using benzothiophene-3-carboxylic acid⁸⁹ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. White solid. MS (ISP): 289.1 ([M+H]⁺).

### Example 25

### (S)-N-(2,6-Dioxopiperidin-3-yl)benzo[b]thiophene-3-carboxamide

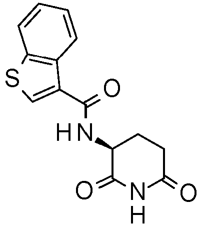

The title compound was obtained in analogy to example 2 using benzothiophene-3-carboxylic acid⁸⁹ in place of 2,3-dihydro-1H-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 289.1 ([M+H]⁺).

### Example 26 (provided for reference)

### (S)-N-((R)-2,6-Dioxopiperidin-3-yl)-1,2,3,4-tetrahydronaphthalene-1-carboxamide

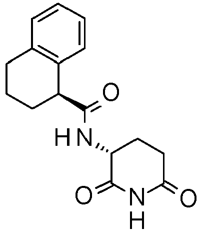

The title compound was obtained in analogy to example 2 using (S)-1,2,3,4-tetrahydronaphthoic acid⁸⁶ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. White solid. MS (ISP): 287.1 ([M+H]⁺).

### Example 27 (provided for reference)

### (S)-N-((S)-2,6-Dioxopiperidin-3-yl)-1,2,3,4-tetrahydronaphthalene-1-carboxamide

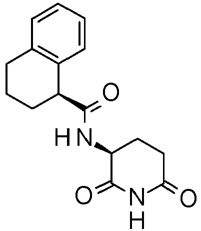

The title compound was obtained in analogy to example 2 using (S)-1,2,3,4-tetrahydronaphthoic acid**^{Error! Bookmark not defined.}** in place of 2,3-dihydro-1H-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 287.1 ([M+H]⁺).

### Example 28

### (S)-N-(2,6-Dioxopiperidin-3-yl)-3,4-dihydroquinoline-1(2H)-carboxamide

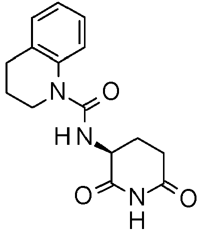

To a stirred suspension of (S)-3-aminopiperidine-2,6-dione hydrochloride⁶² (100 mg) in *N,N-*dimethylformamide (1.5 ml) was added 1,1'-carbonyl-di-(1*H*-imidazole) (128 mg) and the reaction mixture was stirred for 2 hours at room temperature. 1,2,3,4-Tetrahydroquinoline⁹⁰ (125 mg) was added in one portion. The reaction mixture was heated at 80 °C for 3 hours and was then partitioned between ethyl acetate and water. The layers were separated and the aqueous layer was extracted with ethyl acetate (3 x 25 ml). The combined organic layers were washed with brine, dried over anhydrous sodium sulfate, and concentrated *in vacuo.* The resulting residue was purified by reversed phase HPLC to afford (S)-N-(2,6-dioxopiperidin-3-yl)-3,4-dihydroquinoline-1(2H)-carboxamide as a white solid (68 mg, 39%). MS (ISP): 288 ([M+H]⁺).

### Example 29

### (S)-N-(2,6-Dioxopiperidin-3-yl)-2,3-dihydro-1H-pyrrolo[2,3-b]pyridine-1-carboxamide

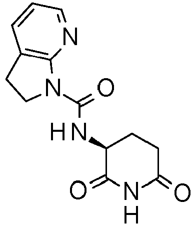

The title compound was obtained in analogy to example 28 using 2,3-dihydro-1H-pyrrolo[2,3-b]pyridine⁹¹ in place of 1,2,3,4-tetrahydroquinoline⁹⁰. Yellow solid. MS (ISP): 275 ([M+H]⁺).

### Example 30

### (S)-N-(2,6-Dioxopiperidin-3-yl)-3,4-dihydro-1,8-naphthyridine-1(2H)-carboxamide

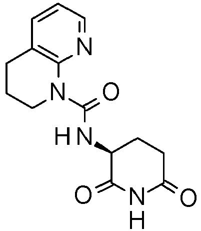

The title compound was obtained in analogy to example 28 using 1,2,3,4-tetrahydro-1,8-naphthyridine⁹² in place of 1,2,3,4-tetrahydroquinoline⁹⁰. White solid. MS (ISP): 289.1 ([M+H]⁺).

### Example 31

### N-[(3S)-2,6-Dioxo-3-piperidyl]chromane-4-carboxamide (MIXTURE OF 2 EPIMERS)

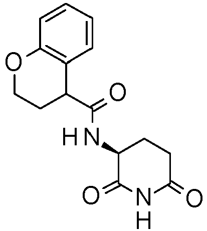

The title compound was obtained in analogy to example 2 using (RS)-chromane-4-carboxylic acid⁹³ in place of 2,3-dihydro-1H-indene-1-carboxylic acid⁷⁵ and (3S)-3-aminopiperidine-2,6-dione hydrochloride⁶² in place of 3-aminopiperidine-2,6-dione⁶⁰. White solid. MS (ISP): 289.2 ([M+H]⁺).

### Example 32

### (4S)-N-[(3S)-2,6-Dioxopiperidin-3-yl]-1,2,3,4-tetrahydroquinoline-4-carboxamide

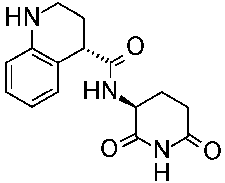

### a) N-((S)-2,6-Dioxoi)ii)eiidin-3-yl)-1,2,3,4-tetrahydroquinoline-4-carboxamide (MIXTURE OF 2 EPIMERS)

The title compound was obtained in analogy to example 2 using (RS)-1,2,3,4-tetrahydroquinoline-4-carboxylic acid ⁹⁴ in place of 2,3-dihydro-1H-indene-1-carboxylic acid⁷⁵ and (3S)-3-aminopiperidine-2,6-dione hydrochloride⁶² in place of 3-aminopiperidine-2,6-dione⁶⁰. White solid. MS (ISP): 288.3 ([M+H]⁺).

### b) (4S)-N-[(3S)-2,6-Dioxopiperidin-3-yl]-1,2,3,4-tetrahydroquinoline-4-carboxamide

The epimers of N-((S)-2,6-dioxopiperidin-3-yl)-1,2,3,4-tetrahydroquinoline-4-carboxamide (160 mg) were separated using chiral HPLC (column: Reprosil Chiral-NR, eluent: heptane/ethanol + NH₄OAc (60:40), pressure: 18 bar; flow rate: 35 ml/min) affording (4*S*)-*N*-[(3*S*)-2,6-dioxopiperidin-3-yl]-1,2,3,4-tetrahydroquinoline-4-carboxamide (57 mg, white solid), retention time = 49 min. MS (ISP): 288.1 ([M+H]⁺).

### Example 33

### (4R)-N-[(3S)-2,6-Dioxopiperidin-3-yl]-1,2,3,4-tetrahydroquinoline-4-carboxamide

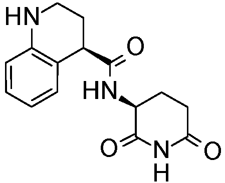

The epimers of N-((,S)-2,6-dioxopiperidin-3-yl)-1,2,3,4-tetrahydroquinoline-4-carboxamide (160 mg) were separated using chiral HPLC (column: Reprosil Chiral-NR, eluent: heptane/ethanol + NH₄OAc (60:40), pressure: 18 bar; flow rate: 35 ml/min) affording (4R)-N-[(3S)-2,6-dioxopiperidin-3-yl]-1,2,3,4-tetrahydroquinoline-4-carboxamide (35 mg, white solid), retention time = 58 min. MS (ISP): 288.1 ([M+H]⁺).

### Example 34

### (R)-N-(2,6-Dioxopiperidin-3-yl)benzofuran-3-carboxamide

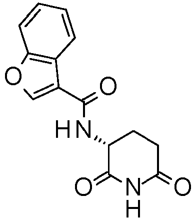

The title compound was obtained in analogy to example 2 using benzofuran-3-carboxylic acid⁹⁵ in place of 2,3-dihydro-1H-indene-1-carboxylic acid ⁷⁵ and (3R)-3-aminopiperidine-2,6-dione hydrochloride⁶⁴ in place of 3-aminopiperidine-2,6-dione⁶⁰. White solid. MS (ISP): 273.1 ([M+H]⁺).

### Example 35

### (R)-N-(2,6-Dioxopiperidin-3-yl)-3,4-dihydroquinoline-1(2H)-carboxamide

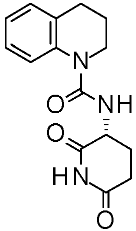

The title compound was obtained in analogy to example 28 using (R)-3-aminopiperidine-2,6-dione hydrochloride⁶⁴ in place of (S)-3-aminopiperidine-2,6-dione hydrochloride⁶². White solid. MS (ISP): 288.1 ([M+H]⁺).

### Example 36

### (R)-N-(2,6-Dioxopiperidin-3-yl)-2,3-dihydro-1H-pyrrolo[2,3-b]pyridine-1-carboxamide

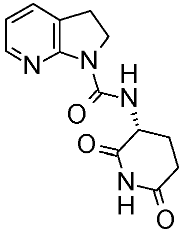

The title compound was obtained in analogy to example 28 using 2,3-dihydro-1H-pyrrolo[2,3-b]pyridine⁹¹ in place of 1,2,3,4-tetrahydroquinoline⁹⁰ and (R)-3-aminopiperidine-2,6-dione hydrochloride⁶⁴ in place of (S)-3-aminopiperidine-2,6-dione hydrochloride⁶². White solid. MS (ISP): 275.1 ([M+H]⁺).

### Example 37

### N-[(3S)-2,6-Dioxo-3-piperidyl]-1,2,3,4-tetrahydroquinoline-4-carboxamide (1:1 MIXTURE OF EPIMERS)

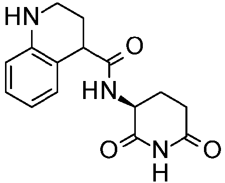

The title compound was obtained in analogy to example 2 using (RS)-1,2,3,4-tetrahydroquinoline-4-carboxylic acid⁹⁴ in place of 2,3-dihydro-1H-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 288.3 ([M+H]⁺).

### Example 38

### N-[(3S)-2,6-Dioxo-3-piperidyl]-1-methyl-3,4-dihydro-2H-quinoline-4-carboxamide (1:1 MIXTURE OF EPIMERS)

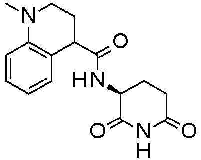

The title compound was obtained in analogy to example 2 using (RS)-1-methyl-1,2,3,4-tetrahydroquinoline-4-carboxylic acid⁹⁶ in place of 2,3-dihydro-1H-indene-1-carboxylic acid⁷⁵ and (3S)-3-aminopiperidine-2,6-dione hydrochloride⁶² in place of 3-aminopiperidine-2,6-dione⁶⁰. White solid. MS (ISP): 302.2 ([M+H]⁺).

### Example 39

### N-[(3R)-2,6-Dioxo-3-piperidyl]-1,2,3,4-tetrahydroquinoline-4-carboxamide (1:1 MIXTURE OF EPIMERS)

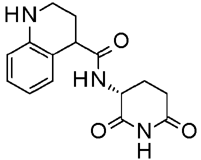

The title compound was obtained in analogy to example 2 using (RS)-1,2,3,4-tetrahydroquinoline-4-carboxylic acid⁹⁴ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. White solid. MS (ISP): 288.3 ([M+H]⁺).

### Example 40

### N-[(3R)-2,6-Dioxo-3-piperidyl]-1-methyl-3,4-dihydro-2H-quinoline-4-carboxamide (1:1 MIXTURE OF EPIMERS)

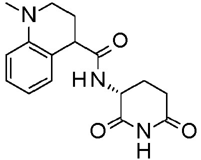

The title compound was obtained in analogy to example 2 using (RS)-1-methyl-1,2,3,4-tetrahydroquinoline-4-carboxylic acid⁹⁶ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. Light red solid. MS (ISP): 302.3 ([M+H]⁺).

### Example 41

### N-[(3R)-2,6-Dioxo-3-piperidyl]chromane-4-carboxamide (1:1 MIXTURE OF EPIMERS)

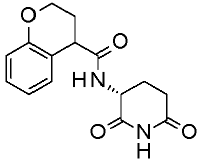

The title compound was obtained in analogy to example 2 using (RS)-chromane-4-carboxylic acid⁹⁷ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. Light red solid. MS (ISP): 289.2 ([M+H]⁺).

### Example 42

### Methyl (S)-3-((2,6-dioxopiperidin-3-yl)carbamoyl)-1H-indole-6-carboxylate

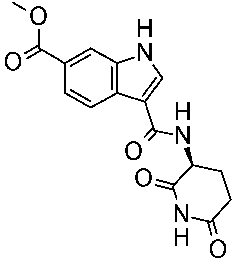

The title compound was obtained in analogy to example 2 using 6-methoxycarbonyl-1H-indole-3-carboxylic acid ⁹⁸ in place of 2,3-dihydro-1H-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 330.1 ([M+H]⁺).

### Example 43

### (4S)-N-[(3R)-2,6-Dioxopiperidin-3-yl]-1,2,3,4-tetrahydroquinoline-4-carboxamide

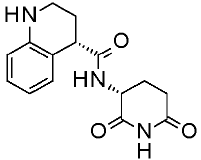

### a) N-[(3R)-2,6-dioxo-3-piperidyl]-1,2,3,4-tetrahydroquinoline-4-carboxamide (MIXTURE OF 2 EPIMERS)

The title compound was obtained in analogy to example 2 using (RS)-1,2,3,4-tetrahydroquinoline-4-carboxylic acid⁹⁴ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. White solid. MS (ISP): 288.3 ([M+H]⁺).

### b) (4S)-N-[(3R)-2,6-Dioxopiperidin-3-yl]-1,2,3,4-tetrahydroquinoline-4-carboxamide

The epimers of *N*-[(3*R*)-2,6-dioxopiperidin-3-yl]-1,2,3,4-tetrahydroquinoline-4-carboxamide (110 mg) from step a were separated using chiral HPLC (column: Reprosil Chiral-NR, eluent: heptane/ethanol + NH₄OAc (60:40), pressure: 18 bar; flow rate: 35 ml/min) to afford *(4S)-N-[(3R)-*2,6-dioxopiperidin-3-yl]-1,2,3,4-tetrahydroquinoline-4-carboxamide (32 mg, white solid), retention time = 39 min. MS (ISP): 288.1 ([M+H]⁺).

### Example 44

### (4R)-N-[(3R)-2,6-Dioxopiperidin-3-yl]-1,2,3,4-tetrahydroquinoline-4-carboxamide

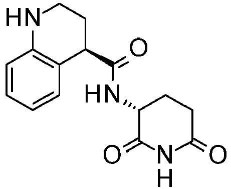

The epimers of *N*-[(3*R*)-2,6-dioxopiperidin-3-yl]-1,2,3,4-tetrahydroquinoline-4-carboxamide (110 mg) were separated using chiral HPLC (column: Reprosil Chiral-NR, eluent: heptane/ethanol + NH₄OAc (60:40), pressure: 18 bar; flow rate: 35 ml/min) to afford (4R)-N-[(3R)-2,6-dioxopiperidin-3-yl]-1,2,3,4-tetrahydroquinoline-4-carboxamide (37 mg, white solid), retention time = 53 min. MS (ISP): 288.1 ([M+H]⁺).

### Example 45 (provided for reference)

### (1R)-N-[(3R)-2,6-Dioxopiperidin-3-yl]-1,2,3,4-tetrahydronaphthalene-1-carboxamide

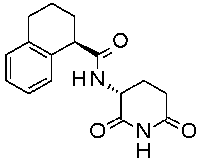

### a) N-[(3R)-2,6-Dioxopiperidin-3-yl]-1,2,3,4-tetrahydronaphthalene-1-carboxamide (MIXTURE OF 2 EPIMERS)

The title compound was obtained in analogy to example 2 using (RS)-1,2,3,4-tetrahydronaphthalene-1-carboxylic acid⁹⁹ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. White solid. MS (ISP): 287.1 ([M+H]⁺).

### b) (1R)-N-[(3R)-2,6-Dioxopiperidin-3-yl]-1,2,3,4-tetrahydronaphthalene-1-carboxamide

The epimers of N-[(3R)-2,6-dioxopiperidin-3-yl]-1,2,3,4-tetrahydronaphthalene-1-carboxamide (250 mg) from step a were separated using chiral HPLC (column: Reprosil Chiral-NR, eluent: heptane/ethanol + NH₄OAc (60:40), pressure: 18 bar; flow rate: 35 ml/min) to afford (1R)-N-[(3R)-2,6-dioxopiperidin-3-yl]-1,2,3,4-tetrahydronaphthalene-1-carboxamide (48 mg, white solid), retention time = 43 min. MS (ISP): 287.1 ([M+H]⁺).

### Example 46 (provided for reference)

### (1R)-N-[(3S)-2,6-Dioxopiperidin-3-yl]-1,2,3,4-tetrahydronaphthalene-1-carboxamide

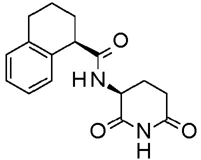

### a) N-[(3S)-2,6-Dioxopiperidin-3-yl]-1,2,3,4-tetrahydronaphthalene-1-carboxamide (MIXTURE OF 2 EPIMERS)

The title compound was obtained in analogy to example 2 using (RS)-1,2,3,4-tetrahydronaphthalene-1-carboxylic acid⁹⁹ in place of 2,3-dihydro-1H-indene-1-carboxylic acid⁷⁵ and (3S)-3-aminopiperidine-2,6-dione hydrochloride⁶² in place of 3-aminopiperidine-2,6-dione⁶⁰. White solid. MS (ISP): 287.2 ([M+H]⁺).

### b) (1R)-N-[(3S)-2,6-Dioxopiperidin-3-yl]-1,2,3,4-tetrahydronaphthalene-1-carboxamide

The epimers of *N*-[(3*S*)-2,6-dioxopiperidin-3-yl]-1,2,3,4-tetrahydronaphthalene-l-carboxamide (210 mg) from step a were separated using chiral HPLC (column: Reprosil Chiral-NR, eluent: heptane/ethanol + NH₄OAc (60:40), pressure: 18 bar; flow rate: 35 ml/min) to afford (1*R*)-*N*-[(3*S*)-2,6-dioxopiperidin-3-yl]-1,2,3,4-tetrahydronaphthalene-1-carboxamide (39 mg, white solid), retention time = 44 min. MS (ISP): 287.1 ([M+H]⁺).

### Example 47

### (S)-N-(2,6-Dioxopiperidin-3-yl)pyrazolo[1,5-a]pyridine-3-carboxamide

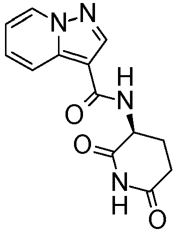

The title compound was obtained in analogy to example 2 using pyrazolo[1,5-a]pyridine-3-carboxylic acid ¹⁰⁰ in place of 2,3-dihydro-1H-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 273.1 ([M+H]⁺).

### Example 48

### (R)-N-(2,6-Dioxopiperidin-3-yl)pyrazolo[1,5-a]pyridine-3-carboxamide

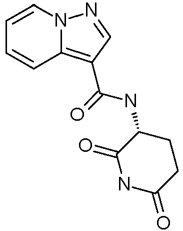

The title compound was obtained in analogy to example 2 using pyrazolo[1,5-a]pyridine-3-carboxylic acid¹⁰⁰ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. White solid. MS (ISP): 273.1 ([M+H]⁺).

### Example 49

### (R)-N-(2,6-Dioxopiperidin-3-yl)-1H-indazole-3-carboxamide

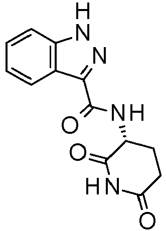

The title compound was obtained in analogy to example 2 using 1H-indazole-3-carboxylic acid¹⁰¹ in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. White solid. MS (ISP): 273.1 ([M+H]⁺).

### Example 50

### (S)-N-(2,6-Dioxopiperidin-3-yl)imidazo[1,5-a]pyridine-3-carboxamide

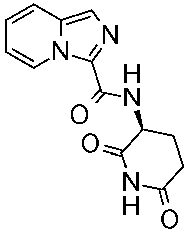

The title compound was obtained in analogy to example 2 using imidazo[1,5-a]pyridine-3-carboxylic acid ¹⁰² in place of 2,3-dihydro-1H-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 273.1 ([M+H]⁺).

### Example 51

### (R)-N-(2,6-Dioxopiperidin-3-yl)imidazo[1,5-a]pyridine-3-carboxamide

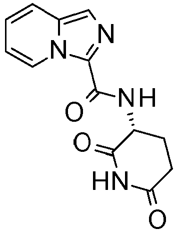

The title compound was obtained in analogy to example 2 using imidazo[1,5-a]pyridine-3-carboxylic acid¹⁰² in place of 2,3-dihydro-1H-indene-1-carboxylic acid⁷⁵ and (3R)-3-aminopiperidine-2,6-dione hydrochloride⁶⁴ in place of 3-aminopiperidine-2,6-dione⁶⁰. White solid. MS (ISP): 273.1 ([M+H]⁺).

### Example 52

### (S)-N-(2,6-Dioxopiperidin-3-yl)indolizine-3-carboxamide

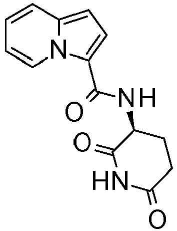

The title compound was obtained in analogy to example 2 using indolizine-3-carboxylic acid¹⁰³ in place of 2,3-dihydro-1*H*-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 272.1 ([M+H]⁺).

### Example 53

### (R)-N-(2,6-Dioxopiperidin-3-yl)indolizine-3-carboxamide

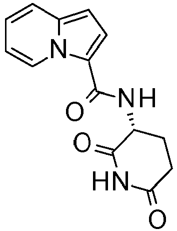

The title compound was obtained in analogy to example 2 using indolizine-3-carboxylic acid¹⁰³ in place of 2,3-dihydro-1*H*-indene-1-carboxylic acid⁷⁵ and (3*R*)-3-aminopiperidine-2,6-dione hydrochloride⁶⁴ in place of 3-aminopiperidine-2,6-dione⁶⁰. White solid. MS (ISP): 272.1 ([M+H]⁺).

### Example 54

### (S)-N-(2,6-Dioxopiperidin-3-yl)imidazo[1,2-a]pyridine-3-carboxamide

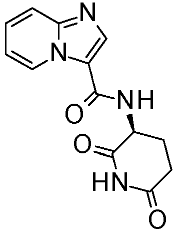

The title compound was obtained in analogy to example 2 using imidazo[1,2-a]pyridine-3-carboxylic acid ¹⁰⁴ in place of 2,3-dihydro-1*H*-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 273.1 ([M+H]⁺).

### Example 55

### (R)-N-(2,6-Dioxopiperidin-3-yl)imidazo[1,2-a]pyridine-3-carboxamide

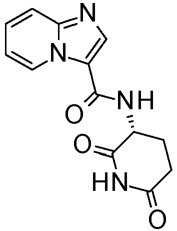

The title compound was obtained in analogy to example 2 using imidazo[1,2-a]pyridine-3-carboxylic acid¹⁰⁴ in place of 2,3-dihydro-1*H*-indene-1-carboxylic acid⁷⁵ and (3*R*)-3-aminopiperidine-2,6-dione hydrochloride⁶⁴ in place of 3-aminopiperidine-2,6-dione⁶⁰. White solid. MS (ISP): 273.1 ([M+H]⁺).

### Example 56

### (S)-N-(2,6-Dioxopiperidin-3-yl)pyrazolo[1,5-a]pyrimidine-3-carboxamide

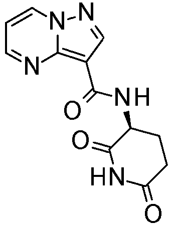

The title compound was obtained in analogy to example 2 using pyrazolo[1,5-a]pyrimidine-3-carboxylic acid ¹⁰⁵ in place of 2,3-dihydro-1*H*-indene-1-carboxylic acid⁷⁵ and (3*S*)-3-aminopiperidine-2,6-dione hydrochloride⁶² in place of 3-aminopiperidine-2,6-dione⁶⁰. White solid. MS (ISP): 274.1 ([M+H]⁺).

### Example 57

### (R)-N-(2,6-Dioxopiperidin-3-yl)pyrazolo[1,5-a]pyrimidine-3-carboxamide

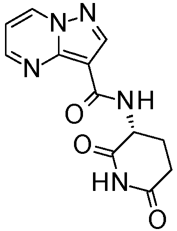

The title compound was obtained in analogy to example 2 using pyrazolo[1,5-a]pyrimidine-3-carboxylic acid¹¹⁵ in place of 2,3-dihydro-1*H*-indene-1-carboxylic acid⁷⁵ and (3*R*)-3-aminopiperidine-2,6-dione hydrochloride⁶⁴ in place of 3-aminopiperidine-2,6-dione⁶⁰. White solid. MS (ISP): 274.1 ([M+H]⁺).

### Example 58

### N-(2,6-Dioxopiperidin-3-yl)-N-methylindoline-1-carboxamide

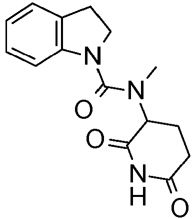

The title compound was obtained in analogy to example 9 using 3-(methylamino)piperidine-2,6-dione⁶⁵ in place of 3-aminopiperidine-2,6-dione⁶⁰. Off-white solid. MS (ISP): 288.1 ([M+H]⁺).

### Example 59

### (S)-N-(2,6-Dioxopiperidin-3-yl)-N-methylindoline-1-carboxamide

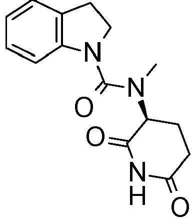

The epimers of *N*-(2,6-dioxopiperidin-3-yl)-*N*-methylindoline-1-carboxamide (120 mg) were separated using chiral HPLC (column: Reprosil Chiral-NR, eluent: heptane/ethanol + NH₄OAc (60:40), pressure: 18 bar; flow rate: 35 ml/min) to afford (*S*)-*N*-(2,6-dioxopiperidin-3-yl)-*N-*methylindoline-1-carboxamide (56 mg, off-white solid), retention time = 12.4 min. MS (ISP): 288.1 ([M+H]⁺).

### Example 60

### (R)-N-(2,6-Dioxopiperidin-3-yl)-N-methylindoline-1-carboxamide

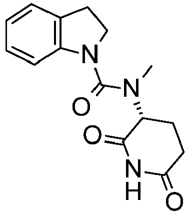

The epimers of *N*-(2,6-dioxopiperidin-3-yl)-*N*-methylindoline-1-carboxamide (120 mg) were separated using chiral HPLC (column: Reprosil Chiral-NR, eluent: heptane/ethanol + NH₄OAc (60:40), pressure: 18 bar; flow rate: 35 ml/min) to afford (*R*)-*N*-(2,6-dioxopiperidin-3-yl)-*N-*methylindoline-1-carboxamide (52 mg, off-white solid), retention time = 15.4 min. MS (ISP): 288.1 ([M+H]⁺).

### Example 61 (provided for reference)

### (S)-N-(2,6-Dioxopiperidin-3-yl)-2-(pyridin-3-yl)acetamide

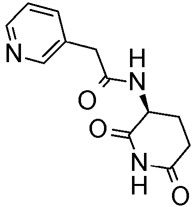

The title compound was obtained in analogy to example 2 using 2-(pyridin-3-yl)acetic acid¹⁰⁶ in place of 2,3-dihydro-1*H*-indene-1-carboxylic acid⁷⁵ and (3*S*)-3-aminopiperidine-2,6-dione ⁶¹ in place of 3-aminopiperidine-2,6-dione⁶⁰. White solid. MS (ISP): 248.1 ([M+H]⁺).

### Example 62

### N-(2,6-Dioxopiperidin-3-yl)-N-methyl-2,3-dihydro-1H-pyrrolo[2,3-b]pyridine-1-carboxamide

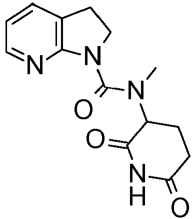

The title compound was obtained in analogy to example 9 using 2,3-dihydro-1*H*-pyrrolo[2,3-*b*]pyridine⁹¹ in place of indoline and 3-(methylamino)piperidine-2,6-dione⁶⁵ in place of 3-aminopiperidine-2,6-dione⁶⁰. Yellow solid. MS (ISP): 289.1 ([M+H]⁺).

### Example 63 (provided for reference)

### (R)-N-(2,6-Dioxopiperidin-3-yl)-2-(pyridin-3-yl)acetamide

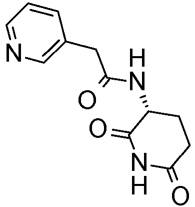

The title compound was obtained in analogy to example 2 using 2-(pyridin-3-yl)acetic acid¹⁰⁶ in place of 2,3-dihydro-1*H*-indene-1-carboxylic acid⁷⁵ and (3*R*)-3-aminopiperidine-2,6-dione⁶³ in place of 3-aminopiperidine-2,6-dione⁶⁰. Off-white solid. MS (ISP): 248.1 ([M+H]⁺).

### Example 64

### (R)-N-(2,6-Dioxopiperidin-3-yl)-3,4-dihydro-1,8-naphthyridine-1(2H)-carboxamide

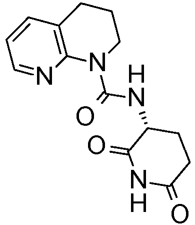

The title compound was obtained in analogy to example 9 using 1,2,3,4-tetrahydro-1,8-naphthyridine⁹² in place of indoline and (*R*)-3-aminopiperidine-2,6-dione⁶³ in place of 3-aminopiperidine-2,6-dione⁶⁰. White solid. MS (ISP): 289.1 ([M+H]⁺).

### Example 65 (provided for reference)

### (S)-2-(4-Acetamidophenyl)-N-(2,6-dioxopiperidin-3-yl)acetamide

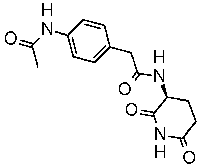

The title compound was obtained in analogy to example 2 using 2-(4-acetamidophenyl)acetic acid¹⁰⁷ in place of 2,3-dihydro-1*H*-indene-1-carboxylic acid⁷⁵ and (3*S*)-3-aminopiperidine-2,6-dione⁶¹ in place of 3-aminopiperidine-2,6-dione⁶⁰. White solid. MS (ISP): 304.1 ([M+H]⁺).

### Example 66 (provided for reference)

### (R)-2-(4-Acetamidophenyl)-N-(2,6-dioxopiperidin-3-yl)acetamide

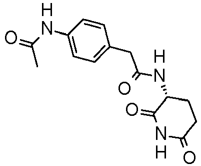

The title compound was obtained in analogy to example 2 using 2-(4-acetamidophenyl)acetic acid¹⁰⁷ in place of 2,3-dihydro-1*H*-indene-1-carboxylic acid⁷⁵ and (3*R*)-3-aminopiperidine-2,6-dione⁶³ in place of 3-aminopiperidine-2,6-dione⁶⁰. Off-white solid. MS (ISP): 304.1 ([M+H]⁺).

### Example 67 (provided for reference)

### (R)-2-(3-Acetamidophenyl)-N-(2,6-dioxopiperidin-3-yl)acetamide

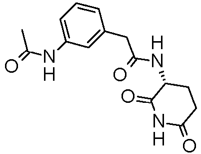

The title compound was obtained in analogy to example 2 using 2-(3-acetamidophenyl)acetic acid¹⁰⁸ in place of 2,3-dihydro-1*H*-indene-1-carboxylic acid⁷⁵ and (3*R*)-3-aminopiperidine-2,6-dione⁶³ in place of 3-aminopiperidine-2,6-dione⁶⁰. White solid. MS (ISP): 304.2 ([M+H]⁺).

### Example 68 (provided for reference)

### (S)-2-(3-Acetamidophenyl)-N-(2,6-dioxopiperidin-3-yl)acetamide

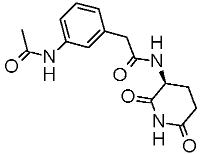

The title compound was obtained in analogy to example 2 using 2-(3-acetamidophenyl)acetic acid¹⁰⁸ in place of 2,3-dihydro-1*H*-indene-1-carboxylic acid⁷⁵ and (3*S*)-3-aminopiperidine-2,6-dione⁶¹ in place of 3-aminopiperidine-2,6-dione⁶⁰. White solid. MS (ISP): 304.1 ([M+H]⁺).

### Example 69

### (S)-N-(2,6-Dioxopiperidin-3-yl)-2-(2-oxoindolin-5-yl)acetamide

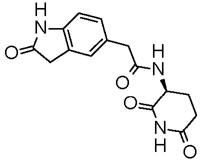

The title compound was obtained in analogy to example 2 using 2-(2-oxoindolin-6-yl)acetic acid¹⁰⁹ in place of 2,3-dihydro-1*H*-indene-1-carboxylic acid⁷⁵ and (3*S*)-3-aminopiperidine-2,6-dione⁶¹ in place of 3-aminopiperidine-2,6-dione⁶⁰. White solid. MS (ISP): 302.1 ([M+H]⁺).

### Example 70

### (R)-N-(2,6-Dioxopiperidin-3-yl)-2-(2-oxoindolin-5-yl)acetamide

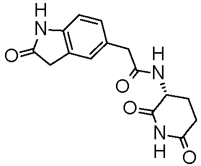

The title compound was obtained in analogy to example 2 using 2-(2-oxoindolin-6-yl)acetic acid¹⁰⁹ in place of 2,3-dihydro-1*H*-indene-1-carboxylic acid⁷⁵ and (3*R*)-3-aminopiperidine-2,6-dione⁶³ in place of 3-aminopiperidine-2,6-dione⁶⁰. White solid. MS (ISP): 302.1 ([M+H]⁺).

### Example 71

### tert-Butyl (S)-3-((2,6-dioxopiperidin-3-yl)carbamoyl)-1-methyl-1,4,6,7-tetrahydro-5H-pyrazolo[4,3-c]pyridine-5-carboxylate

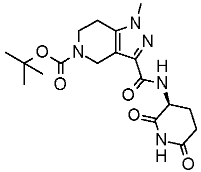

To a mixture of (*S*)-3-aminopiperidine-2,6-dione hydrochloride⁶² (54 mg) and 5-(*tert-*butoxycarbonyl)-1-methyl-4,5,6,7-tetrahydro-1*H*-pyrazolo[4,3-c]pyridine-3-carboxylic acid ¹¹⁰ (102 mg) were added a solution of HATU in *N,N-*dimethylformamide (1.1 ml, 0.358 M) and *N*,*N-*diisopropylethylamine (170 mg). The reaction mixture was shaken at 25 °C for 4 hours. The reaction mixture was then partitioned between water and a 1:1 mixture of ethyl acetate/THF. The organic layer was washed with brine, dried over Na₂SO₄, filtered and concentrated *in vacuo.* The crude material was purified by preparative HPLC followed by lyophilisation to afford *tert*-butyl (*S*)-3-((2,6-dioxopiperidin-3-yl)carbamoyl)-1-methyl-1,4,6,7-tetrahydro-5*H*-pyrazolo[4,3-c]pyridine-5-carboxylate (116 mg, 90.3 %) as a white solid. MS (ISP): 392.4 ([M+H]⁺).

### Example 72

### (S)-N-(2,6-Dioxopiperidin-3-yl)-1-methyl-4,5,6,7-tetrahydro-1H-pyrazolo[4,3-c]pyridine-3-carboxamide

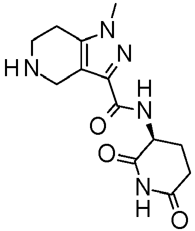

To a stirred solution of *tert*-butyl (*S*)-3-((2,6-dioxopiperidin-3-yl)carbamoyl)-1-methyl-1,4,6,7-tetrahydro-5*H*-pyrazolo[4,3-c]pyridine-5-carboxylate (110 mg) in ethyl acetate (1 ml) was added a solution of 4 M HCl in dioxane (0.5 ml) The reaction mixture was stirred at 0 °C for 2 hours. The product was then collected by filtration, washed with ethyl acetate, and dried to afford (*S*)-*N-*(2,6-dioxopiperidin-3-yl)-1-methyl-4,5,6,7-tetrahydro-1*H*-pyrazolo[4,3-c]pyridine-3-carboxamide (51 mg, 62.3 %) as a white solid. MS (ISP): 292.3 ([M+H]⁺).

### Example 73 (provided for reference)

### (1S)-N-(1-Methyl-2,6-dioxopiperidin-3-yl)-1,2,3,4-tetrahydronaphthalene-1-carboxamide (1:1 MIXTURE OF EPIMERS)

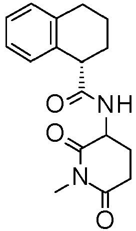

The title compound was obtained in analogy to example 2 using (*S*)-1,2,3,4-tetrahydronaphthoic acid^{**Error! Bookmark not defined**.} in place of 2,3-dihydro-1*H*-indene-1-carboxylic acid⁷⁵ and 3-amino-1-methylpiperidine-2,6-dione hydrochloride¹¹¹ in place of 3-aminopiperidine-2,6-dione⁶⁰. White solid. MS (EI): 300.1 ([M+H]⁺).

### Example 74 (provided for reference)

### (1S)-N-(1-Methyl-2,6-dioxopiperidin-3-yl)-1,2,3,4-tetrahydronaphthalene-1-carboxamide (EPIMER 1)

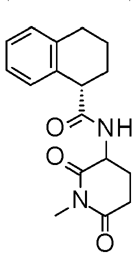

The epimers of (1*S*)-*N*-(1-methyl-2,6-dioxopiperidin-3-yl)-1,2,3,4-tetrahydronaphthalene-1-carboxamide (118 mg) were separated using chiral HPLC (column: Reprosil Chiral-NR, eluent: heptane/ethanol + NH₄OAc (70:30), pressure: 18 bar; flow rate: 35 ml/min) to afford (1*S*)-*N*-(1-methyl-2,6-dioxopiperidin-3-yl)-1,2,3,4-tetrahydronaphthalene-1-carboxamide (EPIMER 1) (29 mg, white solid), retention time = 37 min. MS (EI): 300.1 ([M+H]⁺).

### Example 75 (provided for reference)

### (1S)-N-(1-Methyl-2,6-dioxopiperidin-3-yl)-1,2,3,4-tetrahydronaphthalene-1-carboxamide (EPIMER 2)

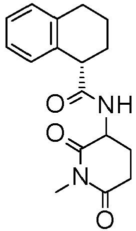

The epimers of (1*S*)-*N*-(1-methyl-2,6-dioxopiperidin-3-yl)-1,2,3,4-tetrahydronaphthalene-1-carboxamide (118 mg) were separated using chiral HPLC (column: Reprosil Chiral-NR, eluent: heptane/ethanol + NH₄OAc (70:30), pressure: 18 bar; flow rate: 35 ml/min) to afford (1*S*)-*N*-(1-methyl-2,6-dioxopiperidin-3-yl)-1,2,3,4-tetrahydronaphthalene-1-carboxamide (EPIMER 1) (29 mg, white solid), retention time = 41 min. MS (EI): 300.1 ([M+H]⁺).
1 Collins et al. Biochem J. 2017, 474(7), 1127-47
2 WO2013020557
3 WO2013063560
4 WO 20131066432013106643n
5 WO2015160845
6 WO2016011906
7 WO2016105518
8 WO2017007612
9 WO2017024318
10 WO2017117473
11 US4558057
12 CN1696127
13 Buckner et al. Mayo Clinic Proceedings, Dowden Health Media, Inc, USA, vol. 74, no. 2, 1 February 1999 (1999-02-01), pages 137-145
14 Huang et al. Zhongguo Yiyao Gongye Zazhi 1993, 24(10), 437-41, 451
15 Xu et al. Zhongguo Yiyao Gongye Zazhi 1992, 23(6), 255-8
16 Hendry et al. Drugs Under Experimental And Clinical Research 1987, 13 (Suppl. 1 ), 77-81
17 WO2008007979
18 CAS No. 1491860-78-6
19 CAS No. 1480842-08-7
20 CAS No. 1466257-08-8
21 CAS No. 1465134-13-7
22 CAS No. 1462991-23-6
23 WO2017197051
24 CAS No. 1487722-61-1
25 CAS No. 1925250-20-9
26 CAS No. 1512181-14-4
27 CAS No. 1466882-97-2
28 CAS No. 1468435-76-8
29 CAS No. 1467495-41-5
30 CAS No. 1480018-59-4
31 CAS No. 1496032-76-8
32 CAS No. 1993422-50-6
33 CAS No. 1869440-20-9
34 CAS No. 1495663-98-3
35 CAS No. 1491860-78-6
36 CAS No. 1485498-58-5
37 CAS No. 1702002-20-7
38 CAS No. 1490092-27
39 CAS No. 1540138-47-3
40 CAS No. 1998850-87-5
41 CAS No. 1966549-05-2
42 CAS No. 1957941-94-4
41 CAS No. 1948185-02-1
44 CAS No. 1925097-89-7
45 CAS No. 1922676-64-9
46 CAS No. 1923778-80-6
47 CAS No. 1560748-55-1
48 CAS No. 1559709-03-3
49 CAS No. 1543949-70-7
50 CAS No. 1541076-23-6
51 CAS No. 1522124-94-2
52 CAS No. 1519516-43-8
53 CAS No. 1492524-17-0
54 CAS No. 1491481-66-3
55 CAS No. 1486311-15-2
56 CAS No. 1467658-22-5
57 CAS No. 1462942-69-3
58 CAS No. 1464846-46-5
59 CAS No. 1466763-16-5
60 CAS No. 2353-44-8
61 CAS No. 29883-25-8
62 CAS No. 25181-50-4
61 CAS No. 673485-72-8
64 CAS No. 1801140-47-5
65 CAS No. 1494409-88-9
66 CAS No. 1268524-70-4
67 Filippakopoulos, P. et al. Nature 2010, 468, 1067-1073
68 CAS No. 202592-23-2
69 CAS No. 26189-59-3
70 J. Chem. Soc., Chem. Commun. 1979, 1180; Org. Synth. 1980, 59, 26-34
71 Yen, H.-C. S., Xu, Q., Chou, D. M., Zhao, Z. & Elledge, S. J. Global Protein Stability Profiling in Mammalian Cells. Science 2008, 322, 918-923, doi:10.1126/science.1160489.
72 Birdsall, B., King, R. W., Wheeler, M. R., Lewis, C. A. Jr, Goode, S. R., Dunlap, R. B. & Roberts, G. C. Anal. Biochem. 1983, 132, 353-361
3 Eftink, Methods Enzymol. 1997, 278, 221-57
74 CAS No. 91531-30-5
75 CAS No. 14381-42-1
76 CAS No. 221352-46-1
77 CAS No. 123-38-6
78 CAS No. 1463830-80-9
79 CAS No. 1495903-25-7
80 CAS No. 496-15-1
81 CAS No. 91393-02-1
2 CAS No. 1493112-18-7
83 CAS No. 1482023-98-2
84 CAS No. 1464854-51-0
85 CAS No. 23357-47-3
86 CAS No. 85977-52-2
87 CAS No. 771-50-6
88 CAS No. 1211516-10-7
89 CAS No. 5381-25-9
90 CAS No. 91-21-4
91 CAS No. 10592-27-5
92 CAS No. 13623-87-5
93 CAS No. 357444-00-9
94 CAS No. 13337-69-4
95 CAS No. 26537-68-8
6 CAS No. 933756-65-1
97 CAS No. 20426-80-6
98 CAS No. 773869-62-8
99 CAS No. 1914-65-4
100 CAS No. 16205-46-2
101 CAS No. 4498-67-3
102 CAS No. 1018587-80-8
103 CAS No. 1259060-82-6
104 CAS No. 6200-60-8
05 CAS No. 25940-35-6
106 CAS No. 501-81-5
107 CAS No. 18699-02-0
108 CAS No. 37777-54-1
109 CAS No. 109737-08-8
110 CAS No. 1306739-66-1
11 CAS No. 1909304-98-8
112 CAS No. 6404-29-1
113 CAS No. 462100-06-7

## Claims

1. A compound of formula I, or a pharmaceutically acceptable salt thereof, 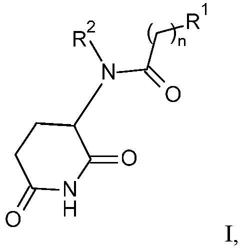 wherein
R¹ is selected from the group consisting of 1,2,3,4-tetrahydroquinolinyl, 1*H*-indolyl, 2,3-dihydro-1*H*-pyrrolo[2,3-b]pyridinyl, 6,7-dihydro-5*H*-cyclopenta[b]pyridinyl, benzofuranyl, benzothiophenyl, chromanyl, indolinyl, and isoindolinyl; each of which is optionally substituted by one or more R⁶,
R² is H, C₁₋₆alkyl or -NC(=O)-C₁₋₆alkyl,
R⁶ is H, C₁₋₆alkyl, -C(=O)O-C₁₋₆alkyl or oxo,
n is 0 or 1;
for use as therapeutically active substance.

2. The compound of formula I, or a pharmaceutically acceptable salt thereof, for use according to claim 1, wherein
R² is H or C₁₋₆alkyl,
R⁶ is H or C₁₋₆alkyl.

3. The compound of formula I, or a pharmaceutically acceptable salt thereof, for use according to claim 1, wherein the compound is selected from the group consisting of
(-)-*N*-((*S*)-2,6-dioxopiperidin-3-yl)-1,2,3,4-tetrahydroquinoline-4-carboxamide,
(+)-*N*-((*S*)-2,6-dioxopiperidin-3-yl)-1,2,3,4-tetrahydroquinoline-4-carboxamide,
*N*-[(3*S*)-2,6-dioxo-3-piperidyl]-1,2,3,4-tetrahydroquinoline-4-carboxamide,
*N*-[(3*R*)-2,6-dioxo-3-piperidyl]-1,2,3,4-tetrahydroquinoline-4-carboxamide,
(*S*)-*N*-((*R*)-2,6-dioxopiperidin-3-yl)-1,2,3,4-tetrahydroquinoline-4-carboxamide,
(4iR)-*N*-[(3*R*)-2,6-dioxopiperidin-3-yl]-1,2,3,4-tetrahydroquinoline-4-carboxamide,
(*S*)-*N*-(2,6-dioxopiperidin-3-yl)-2,3-dihydro-1*H*-pyrrolo[2,3-*b*]pyridine-1-carboxamide,
(*R*)-*N*-(2,6-dioxopiperidin-3-yl)-2,3-dihydro-1*H*-pyrrolo[2,3-b]pyridine-1-carboxamide,
*N*-(2,6-dioxopiperidin-3-yl)-*N*-methyl-2,3-dihydro-1*H*-pyrrolo[2,3-b]pyridine-1-carboxamide,
*N*-(2,6-dioxo-3-piperidyl)-1*H*-indole-3-carboxamide,
*N*-(2,6-dioxo-3-piperidyl)-1-methyl-indole-3-carboxamide,
*N*-(2,6-dioxo-3-piperidyl)indoline-1-carboxamide,
*N*-(2,6-dioxo-3-piperidyl)benzofuran-3-carboxamide,
*N*-(2,6-dioxo-3-piperidyl)benzothiophene-3-carboxamide,
*N*-(2,6-dioxo-3-piperidyl)-*N*-methyl-1*H*-indole-3-carboxamide,
*N*-[(3*S*)-2,6-dioxo-3-piperidyl]-1*H*-indole-3-carboxamide,
*N*-[(3*R*)-2,6-dioxo-3-piperidyl]-1*H*-indole-3-carboxamide,
(*S*)-*N*-(2,6-dioxopiperidin-3-yl)benzofuran-3-carboxamide,
(*R*)-*N*-(2,6-dioxopiperidin-3-yl)benzo[b]thiophene-3-carboxamide,
(*S*)-*N*-(2,6-dioxopiperidin-3-yl)benzo[b]thiophene-3-carboxamide,
*N*-[(3*S*)-2,6-dioxo-3-piperidyl]chromane-4-carboxamide,
(*R*)-*N*-(2,6-dioxopiperidin-3-yl)benzofuran-3-carboxamide,
*N*-[(3*R*)-2,6-dioxo-3-piperidyl]chromane-4-carboxamide,
methyl (*S*)-3-((2,6-dioxopiperidin-3-yl)carbamoyl)-1*H*-indole-6-carboxylate,
*N*-(2,6-dioxopiperidin-3-yl)-*N*-methylindoline-1-carboxamide,
(*S*)-*N*-(2,6-dioxopiperidin-3-yl)-*N*-methylindoline-1-carboxamide,
(*R*)-*N*-(2,6-dioxopiperidin-3-yl)-*N*-methylindoline-1-carboxamide,
*N*-((*S*)-2,6-dioxopiperidin-3-yl)-6,7-dihydro-5*H*-cyclopenta[b]pyridine-5-carboxamide,
*N*-((*R*)-2,6-dioxopiperidin-3-yl)-6,7-dihydro-5*H*-cyclopenta[b]pyridine-5-carboxamide,
*N*-((*R*)-2,6-dioxopiperidin-3-yl)-6,7-dihydro-5*H*-cyclopenta[b]pyridine-7-carboxamide, and
*N*-((*S*)-2,6-dioxopiperldin-3-yl)-6,7-dihydro-5*H*-cyclopenta[b]pyridine-7-carboxamide.

4. A compound of formula I, or a pharmaceutically acceptable salt thereof, 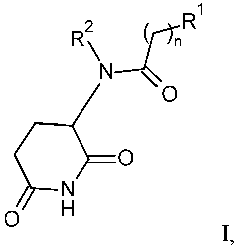 wherein
R¹ is 2,3-dihydro-1*H*-pyrrolo[2,3-b]pyridinyl, optionally substituted by one or more R⁶;
R² is H or C₁₋₆alkyl,
R⁶ is H or C₁₋₆alkyl,
n is 0 or 1.

5. The compound for use according to claim 1 or 2 or the compound according to claim 4, or a pharmaceutically acceptable salt thereof,
wherein
n is 0,
R² is H or C₁₋₆alkyl.

6. The compound for use according to any one of claims 1-3, or a pharmaceutically acceptable salt thereof, wherein R¹ is selected from the group consisting of
i) 1,2,3,4-tetrahydroquinolinyl, and
ii) 1*H*-indolyl.

7. The compound for use according to any one of claims 1-3 or the compound of any one of claims 4-5, or a pharmaceutically acceptable salt thereof, wherein R² is H.

8. The compound for use according to any one of claims 1-3 or the compound of any one of claims 4-5 or 7, or a pharmaceutically acceptable salt thereof, wherein R⁶ is C₁₋₆alkyl.

9. The compound for use or compound of claim 8, wherein R⁶ is Me or propyl.

10. The compound for use according to claim 1 or 2, or a pharmaceutically acceptable salt thereof, selected from the group consisting of
(-)-*N*-((*S*)-2,6-dioxopiperidin-3-yl)-1,2,3,4-tetrahydroquinoline-4-carboxamide,
(+)-*N*-((*S*)-2,6-dioxopiperidin-3-yl)-1,2,3,4-tetrahydroquinoline-4-carboxamide,
(*N*-((*R*)-2,6-dioxopiperidin-3-yl)-6,7-dihydro-5*H*-cyclopenta[b]pyridine-7-carboxamide,
(*R*)-*N*-(2,6-dioxopiperidin-3-yl)benzo[b]thiophene-3-carboxamide,
(*S*)-*N*-(2,6-dioxopiperidin-3-yl)benzo[b]thiophene-3-carboxamide,
(*S*)-*N*-(2,6-dioxopiperidin-3-yl)benzofuran-3-carboxamide,
*N*-((*R*)-2,6-dioxopiperidin-3-yl)-6,7-dihydro-5*H*-cyclopenta[b]pyridine-5-carboxamide,
N-((*S*)-2,6-dioxopiperidin-3-yl)-6,7-dihydro-5*H*-cyclopenta[b]pyridine-5-carboxamide,
*N*-((*S*)-2,6-dioxopiperidin-3-yl)-6,7-dihydro-5H-cyclopenta[b]pyridine-7-carboxamide, and
*N*-[(3*S*)-2,6-dioxo-3-piperidyl]chromane-4-carboxamide.

11. A pharmaceutical composition comprising a compound as defined in any one of claims 1-10, and a therapeutically inert carrier.

## Patentansprüche

1. Verbindung der Formel I oder ein pharmazeutisch unbedenkliches Salz davon, 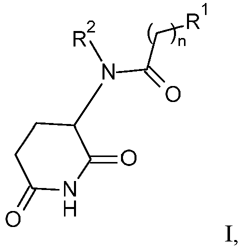 wobei
R¹ ausgewählt ist aus der Gruppe bestehend aus 1,2,3,4-Tetrahydrochinolinyl, 1*H*-Indolyl, 2,3-Dihydro-1*H-*pyrrolo[2,3-b]pyridinyl, 6,7-Dihydro-5*H-*cyclopenta[b]pyridinyl, Benzofuranyl, Benzothiophenyl, Chromanyl, Indolinyl und Isoindolinyl, die jeweils gegebenenfalls durch ein oder mehrere R⁶ substituiert sind,
R² für H, C₁₋₆Alkyl oder -NC(=O)-C₁₋₆Alkyl steht,
R⁶ für H, C₁₋₆Alkyl, -C(=O)₁-C₁-₆Alkyloder Oxo steht,
n für 0 oder 1 steht;
zur Verwendung als therapeutischer Wirkstoff.

2. Verbindung der Formel I oder ein pharmazeutisch unbedenkliches Salz davon zur Verwendung nach Anspruch 1, wobei
R² für H oder C₁₋₆Alkyl steht,
R⁶ für H oder C₁₋₆Alkyl steht.

3. Verbindung der Formel I oder ein pharmazeutisch unbedenkliches Salz davon zur Verwendung nach Anspruch 1, wobei die Verbindung ausgewählt ist aus der Gruppe bestehend aus
(-)-*N*-((*S*)-2,6-Dioxopiperidin-3-yl)-1,2,3,4-tetrahydrochinolin-4-carboxamid,
(+)-*N*-((*S*)-2,6-Dioxopiperidin-3-yl)-1,2,3,4-tetrahydrochinolin-4-carboxamid,
*N*-[(3*S*)-2,6-Dioxo-3-piperidyl]-1,2,3,4-tetrahydrochinolin-4-carboxamid,
*N*-[(3*R*)-2,6-Dioxo-3-piperidyl]-1,2,3,4-tetrahydrochinolin-4-carboxamid,
(*S*)-*N*-((*R*)-2,6-Dioxopiperidin-3-yl)-1,2,3,4-tetrahydrochinolin-4-carboxamid,
(4*R*)-*N*-[(3*R*)-2,6-Dioxopiperidin-3-yl]-1,2,3,4-tetrahydrochinolin-4-carboxamid,
(*S*)-*N*-(2,6-Dioxopiperidin-3-yl)-2,3-dihydro-1*H-*pyrrolo[2,3-b]pyridin-1-carboxamid,
(*R*)-*N*-(2,6-Dioxopiperidin-3-yl)-2,3-dihydro-1*H-*pyrrolo[2,3-b]pyridin-1-carboxamid,
*N*-(2,6-Dioxopiperidin-3-yl)-*N*-methyl-2,3-dihydro-1*H-*pyrrolo[2,3-b]pyridin-1-carboxamid,
*N*-(2,6-Dioxo-3-piperidyl)-1*H*-indol-3-carboxamid,
*N*-(2,6-Dioxo-3-piperidyl)-1-methylindol-3-carboxamid,
*N*-(2,6-Dioxo-3-piperidyl)indolin-1-carboxamid,
*N*-(2,6-Dioxo-3-piperidyl)benzofuran-3-carboxamid,
*N*-(2,6-Dioxo-3-piperidyl)benzothiophen-3-carboxamid,
*N*-(2,6-Dioxo-3-piperidyl)-*N*-methyl-1*H*-indol-3-carboxamid,
*N*-[(3*S*)-2,6-Dioxo-3-piperidyl]-1*H*-indol-3-carboxamid,
*N*-[(3*R*)-2,6-Dioxo-3-piperidyl]-1*H*-indol-3-carboxamid,
(*S*)-*N*-(2,6-Dioxopiperidin-3-yl)benzofuran-3-carboxamid,
(*R*)-*N*-(2,6-Dioxopiperidin-3-yl)benzo[b]thiophen-3-carboxamid,
(*S*)-*N*-(2,6-Dioxopiperidin-3-yl)benzo[b]thiophen-3-carboxamid,
*N*-[(3*S*)-2,6-Dioxo-3-piperidyl]chroman-4-carboxamid,
(*R*)-*N*-(2,6-Dioxopiperidin-3-yl)benzofuran-3-carboxamid,
*N*-[(3*R*)-2,6-Dioxo-3-piperidyl]chroman-4-carboxamid,
(*S*)-3-((2,6-Dioxopiperidin-3-yl)carbamoyl)-1*H*-indol-6-carbonsäuremethylester,
*N*-(2,6-Dioxopiperidin-3-yl)-N-methylindolin-1-carboxamid,
(*S*)-*N*-(2,6-Dioxopiperidin-3-yl)-*N*-methylindolin-1-carboxamid,
(*R*)-*N*-(2,6-Dioxopiperidin-3-yl)-*N*-methylindolin-1-carboxamid,
*N*-((*S*)-2,6-Dioxopiperidin-3-yl)-6,7-dihydro-5*H-*cyclopenta[b]pyridin-5-carboxamid,
*N*-((*R*)-2,6-Dioxopiperidin-3-yl)-6,7-dihydro-5*H-*cyclopenta[b]pyridin-5-carboxamid,
*N*-((*R*)-2,6-Dioxopiperidin-3-yl)-6,7-dihydro-5*H-*cyclopenta[b]pyridin-7-carboxamid und
*N*-((*S*)-2,6-Dioxopiperidin-3-yl)-6,7-dihydro-5*H-*cyclopenta[b]pyridin-7-carboxamid.

4. Verbindung der Formel I oder ein pharmazeutisch unbedenkliches Salz davon, 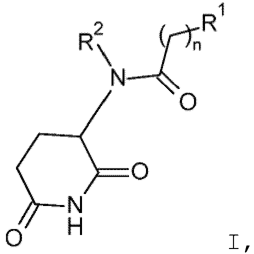 wobei
R¹ für 2,3-Dihydro-1H-pyrrolo[2,3-b]pyridinyl steht, gegebenenfalls substituiert durch ein oder mehrere R⁶,
R² für H oder C₁₋₆Alkyl steht,
R⁶ für H oder C₁₋₆Alkyl steht,
n für 0 oder 1 steht.

5. Verbindung zur Verwendung nach Anspruch 1 oder 2 oder Verbindung nach Anspruch 4 oder ein pharmazeutisch unbedenkliches Salz davon,
wobei
n für 0 steht,
R² für H oder C₁₋₆Alkyl steht.

6. Verbindung zur Verwendung nach einem der Ansprüche 1-3 oder ein pharmazeutisch unbedenkliches Salz davon, wobei R¹ ausgewählt ist aus der Gruppe bestehend aus
i) 1,2,3,4-Tetrahydrochinolinyl und
ii) 1*H*-Indolyl.

7. Verbindung zur Verwendung nach einem der Ansprüche 1-3 oder Verbindung gemäß einem der Ansprüche 4-5 oder ein pharmazeutisch unbedenkliches Salz davon, wobei R² für H steht.

8. Verbindung zur Verwendung nach einem der Ansprüche 1-3 oder Verbindung gemäß einem der Ansprüche 4-5 oder 7 oder ein pharmazeutisch unbedenkliches Salz davon, wobei R⁶ für C₁₋₆Alkyl steht.

9. Verbindung zur Verwendung oder Verbindung gemäß Anspruch 8, wobei R⁶ für Me oder Propyl steht.

10. Verbindung zur Verwendung nach Anspruch 1 oder 2 oder ein pharmazeutisch unbedenkliches Salz davon, ausgewählt aus der Gruppe bestehend aus
(-)-*N*-((*S*)-2,6-Dioxopiperidin-3-yl)-1,2,3,4-tetrahydrochinolin-4-carboxamid,
(+)-*N*-((*S*)-2,6-Dioxopiperidin-3-yl)-1,2,3,4-tetrahydrochinolin-4-carboxamid,
(*N*-((*R*)-2,6-Dioxopiperidin-3-yl)-6,7-dihydro-5*H-*cyclopenta[b]pyridin-7-carboxamid,
(*R*)-*N*-(2,6-Dioxopiperidin-3-yl)benzo[b]thiophen-3-carboxamid,
(*S*)-*N*-(2,6-Dioxopiperidin-3-yl)benzo[b]thiophen-3-carboxamid,
(*S*)-*N*-(2,6-Dioxopiperidin-3-yl)benzofuran-3-carboxamid,
*N*-((*R*)-2,6-Dioxopiperidin-3-yl)-6,7-dihydro-5*H-*cyclopenta[b]pyridin-5-carboxamid,
*N*-((*S*)-2,6-Dioxopiperidin-3-yl)-6,7-dihydro-5*H-*cyclopenta[b]pyridin-5-carboxamid,
*N*-((*S*)-2,6-Dioxopiperidin-3-yl)-6,7-dihydro-5*H-*cyclopenta[b]pyridin-7-carboxamid und
*N*-[(3*S*)-2,6-Dioxo-3-piperidyl]chroman-4-carboxamid.

11. Pharmazeutische Zusammensetzung, umfassend eine Verbindung mit der in einem der Ansprüche 1-10 angegebenen Bedeutung und einen therapeutisch inerten Träger.

## Revendications

1. Composé de formule I, ou sel pharmaceutiquement acceptable de celui-ci, 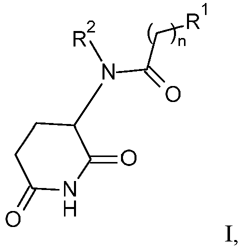 dans laquelle
R¹ est choisi dans le groupe constitué par 1,2,3,4-tétrahydroquinoléinyle, 1H-indolyle, 2,3-dihydro-1H-pyrrolo[2,3-b]pyridinyle, 6,7-dihydro-5H-cyclopenta[b]pyridinyle, benzofuranyle, benzothiophényle, chromanyle, indolinyle, et isoindolinyle ; chacun desquels étant éventuellement substitué par un ou plusieurs R⁶,
R² est H, C₁₋₆alkyle ou -NC(=O)-C₁₋₆alkyle,
R⁶ est H, C₁₋₆alkyle, -C(=O)O-C₁₋₆alkyle ou oxo,
n est 0 ou 1 ;
pour une utilisation comme substance thérapeutiquement active.

2. Composé de formule I, ou sel pharmaceutiquement acceptable de celui-ci, pour une utilisation selon la revendication 1, dans laquelle
R² est H ou C₁₋₆alkyle,
R⁶ est H ou C₁₋₆alkyle.

3. Composé de formule I, ou sel pharmaceutiquement acceptable de celui-ci, pour une utilisation selon la revendication 1, où le composé est choisi dans le groupe constitué par
(-)-N-((S)-2,6-dioxopipéridin-3-yl)-1,2,3,4-tétrahydroquinoline-4-carboxamide,
(+)-N-((S)-2,6-dioxopipéridin-3-yl)-1,2,3,4-tétrahydroquinoline-4-carboxamide,
N-[(3S)-2,6-dioxo-3-pipéridinyl]-1,2,3,4-tétrahydroquinoline-4-carboxamide,
N-[(3R)-2,6-dioxo-3-pipéridinyl]-1,2,3,4-tétrahydroquinoline-4-carboxamide,
(S)-N-((R)-2,6-dioxopipéridin-3-yl)-1,2,3,4-tétrahydroquinoline-4-carboxamide,
(4R)-N-[(3R)-2,6-dioxopipéridin-3-yl]-1,2,3,4-tétrahydroquinoline-4-carboxamide,
(S)-N-(2,6-dioxopipéridin-3-yl)-2,3-dihydro-1H-pyrrolo[2,3-b]pyridine-1-carboxamide,
(R)-N-(2,6-dioxopipéridin-3-yl)-2,3-dihydro-1H-pyrrolo[2,3-b]pyridine-1-carboxamide,
N-(2,6-dioxopipéridin-3-yl)-N-méthyl-2,3-dihydro-1H-pyrrolo[2,3-b]pyridine-1-carboxamide,
N-(2,6-dioxo-3-pipéridinyl)-1H-indole-3-carboxamide,
N-(2,6-dioxo-3-pipéridinyl)-1-méthyl-indole-3-carboxamide,
N-(2,6-dioxo-3-pipéridinyl)indoline-1-carboxamide,
N-(2,6-dioxo-3-pipéridinyl)benzofurane-3-carboxamide,
N-(2,6-dioxo-3-pipéridinyl)benzothiophéne-3-carboxamide,
N-(2,6-dioxo-3-pipéridinyl)-N-méthyl-1H-indole-3-carboxamide,
N-[(3S)-2,6-dioxo-3-pipéridinyl]-1H-indole-3-carboxamide,
N-[(3R)-2,6-dioxo-3-pipéridinyl]-1H-indole-3-carboxamide,
(S)-N-(2,6-dioxopipéridin-3-yl)benzofurane-3-carboxamide,
(R)-N-(2,6-dioxopipéridin-3-yl)benzo[b]thiophéne-3-carboxamide,
(S)-N-(2,6-dioxopipéridin-3-yl)benzo[b]thiophéne-3-carboxamide,
N-[(3S)-2,6-dioxo-3-pipéridinyl]chromane-4-carboxamide,
(R)-N-(2,6-dioxopipéridin-3-yl)benzofurane-3-carboxamide,
N-[(3R)-2,6-dioxo-3-pipéridinyl]chromane-4-carboxamide,
(S)-3-((2,6-dioxopipéridin-3-yl)carbamoyl)-1H-indole-6-carboxylate de méthyle,
N-(2,6-dioxopipéridin-3-yl)-N-méthylindoline-1-carboxamide,
(S)-N-(2,6-dioxopipéridin-3-yl)-N-méthylindoline-1-carboxamide,
(R)-N-(2,6-dioxopipéridin-3-yl)-N-méthylindoline-1-carboxamide,
N-((S)-2,6-dioxopipéridin-3-yl)-6,7-dihydro-5H-cyclopenta[b]pyridine-5-carboxamide,
N-((R)-2,6-dioxopipéridin-3-yl)-6,7-dihydro-5H-cyclopenta[b]pyridine-5-carboxamide,
N-((R)-2,6-dioxopipéridin-3-yl)-6,7-dihydro-5H-cyclopenta[b]pyridine-7-carboxamide, et
N-((S)-2,6-dioxopipéridin-3-yl)-6,7-dihydro-5H-cyclopenta[b]pyridine-7-carboxamide.

4. Composé de formule I, ou sel pharmaceutiquement acceptable de celui-ci, 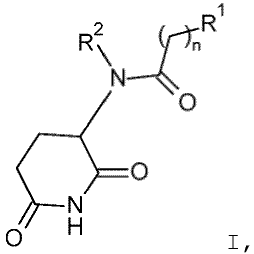 dans laquelle
R¹ est 2,3-dihydro-1H-pyrrolo[2,3-b]pyridinyle, éventuellement substitué par un ou plusieurs R⁶ ;
R² est H ou C₁₋₆alkyle,
R⁶ est H ou C₁₋₆alkyle,
n est 0 ou 1.

5. Composé pour utilisation selon la revendication 1 ou 2 ou composé selon la revendication 4, ou sel pharmaceutiquement acceptable de celui-ci,
où
n est 0,
R² est H ou C₁₋₆alkyle.

6. Composé pour utilisation selon l'une quelconque des revendications 1 à 3, ou sel pharmaceutiquement acceptable de celui-ci, où R¹ est choisi dans le groupe constitué par
i) 1,2,3,4-tétrahydroquinoléinyle, et
ii) 1H-indolyle.

7. Composé pour utilisation selon l'une quelconque des revendications 1-3 ou composé selon l'une quelconque des revendications 4-5, ou sel pharmaceutiquement acceptable de celui-ci, où R² est H.

8. Composé pour utilisation selon l'une quelconque des revendications 1-3 ou composé selon l'une quelconque des revendications 4-5 ou 7, ou sel pharmaceutiquement acceptable de celui-ci, où R⁶ est C₁₋₆alkyle.

9. Composé pour utilisation selon la revendication 8, dans lequel R⁶ est Me ou propyle.

10. Composé pour utilisation selon la revendication 1 ou 2, ou sel pharmaceutiquement acceptable de celui-ci, choisi dans le groupe constitué par
(-)-N-((S)-2,6-dioxopipéridin-3-yl)-1,2,3,4-tétrahydroquinoline-4-carboxamide,
(+)-N-((S)-2,6-dioxopipéridin-3-yl)-1,2,3,4-tétrahydroquinoline-4-carboxamide,
(N-((R)-2,6-dioxopipéridin-3-yl)-6,7-dihydro-5H-cyclopenta[b]pyridine-7-carboxamide,
(R)-N-(2,6-dioxopipéridin-3-yl)benzo[b]thiophéne-3-carboxamide,
(S)-N-(2,6-dioxopipéridin-3-yl)benzo[b]thiophéne-3-carboxamide,
(S)-N-(2,6-dioxopipéridin-3-yl)benzofurane-3-carboxamide,
N-((R)-2,6-dioxopipéridin-3-yl)-6,7-dihydro-5H-cyclopenta[b]pyridine-5-carboxamide,
N-((S)-2,6-dioxopipéridin-3-yl)-6,7-dihydro-5H-cyclopenta[b]pyridine-5-carboxamide,
N-((S)-2,6-dioxopipéridin-3-yl)-6,7-dihydro-5H-cyclopenta[b]pyridine-7-carboxamide, et
N-[(3S)-2,6-dioxo-3-pipéridinyl]chromane-4-carboxamide.

11. Composition pharmaceutique comprenant un composé selon l'une quelconque des revendications 1 à 10 et un support thérapeutiquement inerte.
